# EUROPEAN PATENT SPECIFICATION

(11) **EP 1 774 001 B1**
(45) Date of publication and mention of the grant of the patent: **22.08.2012**
(21) Application number: 05762794.5
(22) Date of filing: 22.06.2005
(51) Int. Cl.: A61K 38/36, C07K 14/745

(54) **Mammalian Tissue Factor obtained from plants and applications of the same**
Aus Pflanzen erhaltener Säugetier-spezifischer Gewebefaktor und dessen Anwendungen
Facteur tissulaire de mammifères obtenus à partir de plantes et applications

(30) Priority: 25.06.2004 US 583187 P
(43) Date of publication of application: 18.04.2007
(73) Proprietor: Altor BioScience Corporation, Miramar, FL 33025-3958 (US)
(72) Inventor: NEGROUK, Valentin, Weston, FL 33326 (US); WONG, Hing, C., Weston, FL 33322 (US); TAYLOR, Dean, Weston, FL 33321 (US); HAN, Kai-ping, Miramar, FL 33025 (US)
(74) Representative: Hiebl, Inge Elisabeth
(86) International application number: PCT/US2005/022766
(87) International publication number: WO 2006/004675

(56) References cited:
- EP-A- 0 787 802
- WO-A-01/77357
- WO-A-91/02066
- WO-A-99/58699
- US-A- 5 346 991
- US-A1- 2003 221 223
- US-B1- 6 261 803
- NEGROUK V ET AL: "Highly efficient transient expression of functional recombinant antibodies in lettuce" PLANT SCIENCE, LIMERICK, IE, vol. 169, no. 2, August 2005 (2005-08), pages 433-438, XP004965463 ISSN: 0168-9452

## Description

### FIELD OF THE INVENTION

The invention generally relates to the production and use of a recombinant mammalian tissue factor obtained from lettuce. Tissue factor is a key component of blood coagulation cascade. The invention has a wide spectrum of useful applications including providing highly active tissue factor for use in the prevention or treatment of bleeding and wound healing.

### BACKGROUND

There is general acknowledgment that an important step in wound treatment is to reduce or eliminate breeding. That step usually requires blood coagulation not only to stem the loss of blood but to initiate wound closure and healing. More generally, blood coagulation is thought to help restore homeostasis and promote faster and more efficient wound healing. See generally L. Stryer, Biochemistry, 3rd Ed, W.H. Freeman Co., New York; A.G. Gilman et al., The Pharmacological Basis of Therapeutics, 9th Edition, McGraw Hill Inc., New York, pp. 1341-1359; and Mann, K.G. et al. (1992) Semin. Hematol. 29:213.

Biological pathways that promote blood coagulation have been reported. These include an intrinsic and an extrinsic pathway. See S.L, Rapaport and L.V.M. Rao (1995) Throm. Haemost. 74: 7; and Stryer, L. *supra*

The extrinsic pathway of blood coagulation is thought to be triggered by exposure to a blood coagulation factor called Tissue Factor (TF). More specifically, the extrinsic (TF) pathway is thought to start when blood comes in contact with TF. Exposure of flowing blood to TF initiates clotting. In the presence of calcium, TF and FVIIa form a complex (TF-FVIIa) which is thought to catalyze activation of other "downstream" blood factors FX and FIX. FXa and Factor Va form the "prothrombinase" complex" which is believed to convert prothrombin to thrombin. Thrombin helps form the blood clot. See, e.g., Davie, E.W. et al. (1991) Biochem. 30: 10363; Nemerson, Y. (1988) Blood 71:1, Rand, M.D. et al. (1996) Blood 88:1; and Monroe, D.M. et al. (1994) Brit. J. of Haemot. 88: 364.

The structure and function of tissue factor have been disclosed. See, e.g., USP Nos. 5,110,730; 5,298,599; and 5,622,931. In particular, the nucleic acid and amino acid sequence of human tissue factor has been reported. See GenBank Accession Nos. J02846 (gene sequence) and BC011029 (cDNA, mRNA and protein sequences). It is generally believed that TF requires an association with certain membrane components, typically phospolipids to achieve optimal activity. See Williams Hematology, 5th Ed. (Beutler, E. et al. Eds.) McGraw-Hill, Inc., Health Professions Div., New York.

Methods for determining how quickly blood clots are known. These methods are useful, for instance, for identifying blood factor deficiencies in patients. One standard method (called the prothrombin time (PT) test) uses calcified blood plasma as a starting reagent. Subsequently, the plasma is combined with thromboplastin or TF. Clotting times are then measured. According to a standard method, clot reaction can be initiated by addition of lipidated recombinant human TF (rhTF) in the presence of Ca⁺⁺. An automated coagulation timer can be used to determine the clot time. See e.g., Miletich, JP (1995) in Prothrombin Time (Chapter L33) Williams Hematology, 5th Ed., supra, pages L82-L84.

Although some microorganisms have been used to make rhTF, it is known that such material is not always useful. For instance, there is general recognition that optimal blood clotting activity is achieved with purified TF or rhTF protein preparations are combined with certain phospholipids in a process referred to "relipidation" of the rhTF. This extra processing step adds to the cost of making and using rhTF in many settings. Consistent lipidation of TF is believed to be a critical factor to the success of many *in vitro* blood clotting assays.

There have been efforts to use plants to make certain heterologous proteins. See e.g., PCT/US02/23624 to Bascomb, N. et al.; PCT/US02/17927 to Hall, G. et al; Daniell, H et al. (2001) Trends in Plant Science, Vol. 6: 219

In particular, certain plants have been reported to be an attractive alternative to microbial and animal cell based protein production systems. There are many reports concerning the benefits of producing proteins in plants. These include straightforward purification procedures and the ability to "farm" the protein on a large scale using traditional agronomic practices.

WO 99/58699 relates to a coagulation factor capable of eliciting an activation response in the human blot clotting pathway comprising a blood clotting protein derived from a transgenic plant, said transgenic plant comprising and expressing a DNA sequence coding for a blood clotting factor protein, said blood clotting protein extracted from said transgenic plant being capable of eliciting an activation response in a human blood coagulation pathway upon administration of the coagulation factor.

WO 01/77357 discloses a non-integrating chromosomal vector comprising an exogenous nucleic acid sequence that is transmitted through the male gametogenesis in each generation and which provides for the production of an exogenic substance.

US 5,346,991 discloses a tissue factor protein mutant capable of neutralizing the ability of endogenous tissue factor to induce coagulation.

EP 0 787 802 discloses a tissue factor protein essentially free of substance with which it is associated in its in vivo physiological milieu and a pharmaceutical composition useful for the treatment of coagulation disorder comprising said tissue factor protein.

WO 91/02066 discloses a process for the production in a plant host of heterologous protein material which is to be excreted from the cells in which it is produced by growing plants or plant cells that by means of genetic manipulation, optionally of an ancestor, using a recombinant polynucleotide have been provided with the genetic information which is required to allow the plant host to express the heterologous protein material and excrete it from the cells in which it is formed, the genetic information introduced into the host cell comprising an expression cassette that is functional in the host and contains at least a gene coding for the mature protein material, directly preceded by genetic information coding for a plant signal peptide that is functional in the host.

J. Kapusta et al., "A plant-derived edible vaccine against hepatitis B virus", THE FASEB JOURNAL, Vol. 13, pages 1796-1799 (1999) discloses a plant-derived edible vaccine against hepatitis B virus.

Notwithstanding these benefits, it is not always clear whether all heterologous proteins can be made in plants efficiently. In particular, it is not certain whether plants have the capacity to fold and modify all such proteins following translation such that the protein is soluble and biologically functional. Preferred codon usage necessary to achieve reasonable levels of expression is not always known for many such proteins. Plant membrane components that may impact protein production or function are usually not completely understood. In some cases, plants may suppress the production of certain heterologous proteins by a process referred to as silencing.

Notwithstanding these difficulties, it would be desirable to have transgenic plants that can express tissue factor. It would be further desirable to have plant-derived recombinant tissue factor (rhTF) in which the need for relipidation has been minimized and preferably eliminated. It would also be desirable to have vectors for making the plants as well as compositions and methods that use the plant-derived rhTF to prevent or treat wounds in a patient

### SUMMARY OF THE INVENTION

The invention relates to a recombinant mammalian tissue factor obtained from a transgenic lettuce (*Lactucua sativa*) and comprising at least one plant-specific glycan wherein the recombinant mammalian tissue factor can be used as a coagulant without significant artificial lipidation, and wherein the plant-specific glycan lacks at least one and preferably both of terminal galactose and terminal acetylneuraminic acid (NeuAc; sialic acid) group and includes at least one of and preferably both of α(1,3) fucose and β(1,2) xylose group. The invention has a wide spectrum of useful applications including use in diagnostic assays and as an agent for promoting wound healing.

We have found that it is possible to express TF in lettuce and to obtain functional TF protein that requires minimal if any relipidation. That is, we have discovered that lettuce can supply cellular components that help promote TF function. Without wishing to be bound to theory, it is believed that lettuce membranes can provide component molecules that can substitute for those with which TF naturally associates (or associates synthetically). This observation is surprising in that there has not been universal acknowledgment that Lettuce can express functional TF that requires minimal if any lipidation to achieve significant blood clotting activity.

These and other surprising features of the invention provide substantial advantages. For example, and in accord with the present invention, it is now possible to express functional TF in lettuce in a fashion that is more economical than prior methods of using fermentation or bioreactor systems to express TF in microbes. In addition, and in accord with methods provided by the invention, it is now possible to "farm" functional TF on a relatively large scale, i.e., to utilize standard technologies for transforming, harvesting, and processing transgenic lettuce to make the protein. Moreover, the TF obtained from such methods requires minimal if any relipidation to make functional protein. This invention provides several specific advantages including lowering the cost of obtaining functional TF and providing for TF that has more uniforrn activity from batch to batch. These advantages, in turn, will help provide better and more efficient treatment to prevent blood loss or promote wound healing and further, for use in existing diagnostic assays such as those intended to monitor blood clotting *in vitro.*

Accordingly, the invention provides for a recombinant mammalian tissue factor obtained from a transgenic lettuce (*Lactucua sativa*) and comprising at least one plantspecific glycan wherein the recombinant mammalian tissue factor can be used as a coagulant without significant artificial lipidation, and wherein the plant-specific glycan lacks at least one and preferably both of terminal galactose and terminal acetylneuraminic acid (NeuAc; sialic acid) group and includes at least one of and preferably both of α(1,3) fucose and β(1,2) xylose group. Typically, the recombinant tissue factor
does not require any significant relipidation to achieve useful activity in a standard prothrombin time (PT) assay. Examples of such PT assays are described below.

More particular recombinant mammalian tissue factor in accord with the invention include an operatively linked plantor mammal-derived signal peptide.

The recombinant mammalian tissue factor of the invention includes at least one plant-specific glycan. Preferably, that glycan is covalently linked to the protein preferably through one or more intervening carbohydrate groups. Examples of such plant-specific glycans are an α(1,3) fucose group and a β(1,2) xylose group. Still other plant-derived recombinant mammalian tissue factor proteins and functional fragments thereof do not usually include carbohydrates that are indicative of expression in animal cells e.g., one or more of a terminal galactose or sialic-acid group. By "terminal" is meant that the animal specific carbohydrate is typically positioned at or near the end of the glycan structure bound to the protein.

The recombinant mammalian tissue factor can be provided in the form of a crude plant extract or in a more purified form as needed to suit a particular invention objective. Thus in one embodiment, the tissue factor is provided as a substantially pure preparation.

As discussed, such recombinant mammalian tissue factor provided by the invention does not require significant relipidation to achieve optimal biological activity in a wide variety of assays including those that monitor blood clotting such as a PT test.

The disclosure further describes a variety of suitable transgenic lettuce plants that include transformed plant cells that express the recombinant mammalian tissue factor or functional fragment thereof. Typically transformed plant cells included within the plant comprise at least one vector that expresses the protein as disclosed herein. Nearly any appropriate monocot or dicot can be transformed by the methods of the invention such as those representative plants described below. Also contemplated are progeny plants, cuttings (sometimes called "slips"), cultivars, or a seed obtained from the tansgenic plant. Preferably, such a progeny plant, cutting, slip, cultivar, or seed includes the vector expressing the mammalian tissue factor or fragment.

Such transgenic lettuce plants are generally prepared by administering one or more suitable vectors that encode tissue factor or a functional fragment thereof. Thus in another aspect, there is described an expression vector that includes in one embodiment: (a) a first promoter operable in a plant; (b) an expressible nucleic acid sequence encoding a mammalian tissue factor protein or functional fragment thereof, wherein the nucleic acid sequence is operatively associated with the promoter; and (c) a termination sequence operatively associated with the nucleic acid sequence. Methods for making and using such vectors are described below.

Further provided is a crude extract of the transgenic lettuce plant that includes the recombinant tissue factor (rhTF).

More particular extracts include the protein expressed by the plant suspended in an aqueous fraction. That fraction can be made by one or a combination of standard extraction or purification strategies such as those mentioned below. The crude extract is readily prepared by partially or completely disrupting cells of the plant (or a portion thereof such as the leaves, flowers, etc.) in the presence of an aqueous solution and removing any insoluble components by low speed centrifugation, gravity and the like to make the crude extract.

The crude extract prepared in accord with the invention provides distinct benefits. For instance, it includes functional rhTF that can be used in a variety of applications without the need for lipidation or further purification. That is, the crude extract can be used as a "stand alone" composition (coagulant) or it can be combined with other compositions (e.g., pharmaceutically acceptable vehicles, additives, excipients and the like) to suit an intended use. As will be apparent, the crude extract can be prepared in relatively large quantities, thereby assisting its widespread use in which substantial amounts of rhTF are needed in which the preparation must have essentially consistent biological activity.

Accordingly, and in another aspect the invention features a coagulant or composition that includes such an agent that comprises the recombinant mammalian tissue factor produced by the transgenic lettuce plant

Described is a method of making the transgenic lettuce plant disclosed herein. The method includes at least one and preferably all of the following steps:
i) administering to a lettuce plant or plant tissue at least one expression vector comprising (a) a first promoter operable in a plant, (b) an expressible nucleic acid sequence encoding a mammalian tissue factor protein, wherein the nucleic acid sequence is operatively associated with the promoter; and (c) a termination sequence operatively associated with the nucleic acid sequence; and
ii) incubating the plant or plant tissue under conditions conducive to expressing the mammalian tissue factor protein,
   thereby making the transgenic plant. This embodiment is well-suited for a variety of transformation applications including those involving conventional particle bombardment, whisker transformation, electroporation, and protoplast transformation.

If desired, a more particular strategy for making the transgenic plants can be followed as generally provided by these steps:
i) administering to a suitable *Agrobacterium* strain at least one replicable (in *Agrobacterium*) expression vector comprising (a) a first promoter operable in a plant, (b) an expressible nucleic acid sequence encoding a mammalian tissue factor protein,
   wherein the nucleic acid sequence is operatively associated with the promoter; and (c) a termination sequence operatively associated with the nucleic acid sequence; and
ii) incubating the bacterium with a lettuce plant or plant tissue under conditions conducive to transforming the plant or plant tissue with the vector to make the transgenic plant. As will be appreciated, this part is well suited for applications in which use of *Agrobacterium*-mediated transformation is desired.

Further disclosed are pharmaceutically useful forms of the rhTF suitable for the uses described herein. Included are aqueous suspensions, lyophilized powders, dry powder or liquid aerosol sprays, topical applications such as liquids or creams. Also included are forms in which the rhTF is provided in the form of a solid support, typically an absorbent (or adsorbent) that includes the rhTF disclosed herein. In this aspect, the absorbent (or adsorbent) holds the protein for use in treatments in which more focused and/or prolonged application of the rhTF is needed. Such a solid support can include an adhesive support adapted to apply the absorbent (or adsorbent). Also envisioned is a wrapper container that includes the adhesive support and is preferably essentially impervious to water, air, and contaminants.

It is an object of the present invention to provide crude plant extracts and substantially purified preparations thereof for use in delivering rhTF to a subject in need of blood clotting. That is, it has been found that the rhTF can be used as a coagulant without significant artificial lipidation. In contrast, most commercial sources of rhTF are produced in a way that requires substantial lipidation to achieve good activity.

Accordingly, it is a further object of the invention to provide a use for promoting wound healing in a subject. In one embodiment, the use includes contacting that subject with a therapeutically effective amount of the coagulant. In a further invention object, there is provided a use for promoting angiogenesis in a subject. Preferably, the use includes contacting the subject with a therapeutically effective amount of the coagulant disclosed herein. It is another invention object to provide a use for promoting vascular remodeling in a subject. In one embodiment, the use includes contacting the subject with a therapeutically effective amount of the coagulant described herein.

The subject is preferably a mammal and usually a human patient in need of such treatment. Although these uses will find use in a variety of settings, preferred practice will often involve situations in which rapid cessation of blood flow is useful. Such situations include those often associated with a medical, public health, or military emergency such as those associated with a hospital (ER) room, disaster area, or battle field and can also include veterinary applications.

### BRIEF DESCRIPTION OF THE DRAWINGS

Figures 1A-B are drawings showing the amino acid sequence of human tissue factor (bold) with corresponding signal peptide (underlined) preferred vector (1A). Also shown as Fig. 1B is the sequence of human tissue factor (bold) with tomato subtilase signal peptide (underlined). Putative glycosylation sites are shown (double underline).
Figure 2 is a drawing showing a physical map of a preferred plant transformation vector (pSUNP12).
Figure 3 is a photograph of a NuPAGE Bis-tris gel (12%) of purified plant rhTF. Lane 1 - molecular weight marker; lane 2 - purified lettuce full length (263 aa) rhTF; lane 3 - purified *E.coli* 243aa rhTF.
Figure 4 is a graph showing results of a PT assay of rhTF₂₄₃ (*E. coli* derived, purified and relipidated), lettuce-derived rhTF (purified and relipidated) and crude extract of lettuce-derived rhTF at different doses.
Figure 5 is a graph with results of a PT assay showing the effect of anti-TF antibody, Sunol-cM36, on *E*. *coli* rhTF243 and lettuce-derived rhTF.
Figure 6A-E are photographs of wounds after treatment with water (dsH2O; 6A), PBS (6B), *E. coli* TF (243; 6C), purified plant TF (6D), and plant crude extract TF (6E).
Figure 7A-F is a drawing showing the complete nucleotide sequence of the vector represented schematically in Figure 2.

### DETAILLED DESCRIPTION

As discussed, described are transgenic lettuce plants that express mammalian and preferably human recombinant tissue factor (rhTF) as well as methods for making and using that rhTF. Such plant-derived protein has been found to greatly assist blood clotting without the need to perform any relipidation steps. The invention has a wide spectrum of useful applications including use as a coagulant, for promoting wound healing and related functions such as angiogenesis and in diagnostic assays (e.g., PT, aPTT assays).

As also discussed, the invention provides, in one aspect, rhTF that is obtained from a transgenic lettuce plant that expresses the protein. By the phrase "tissue factor protein" is meant a mammalian tissue factor protein, preferably mature human tissue factor protein as represented by the amino acid sequence shown below in Table IA. The information can also be found at Genbank Accession No. BC011029. See the National Center for Biotechnology Information (NCBI)- Genetic Sequence Data Bank (Genbank), National Library of Medicine, 38A, 8N05, Rockville Pike, Bethesda, MD 20894. See generally Benson, D.A. et al. (1997) Nucl. Acids. Res. 25: 1 for a description of Genbank.

**TABLE I**

| A-Mature Human Tissue Factor Sequence (1-263) (SEQ ID NO: 4): |
|---|
| |

| B. Human Tissue Factor Signal Peptide Sequence (SEQ ID NO: 5) |
|---|
| METPAWPRVPRPETAVARTLLLGWVFAQVAGA |

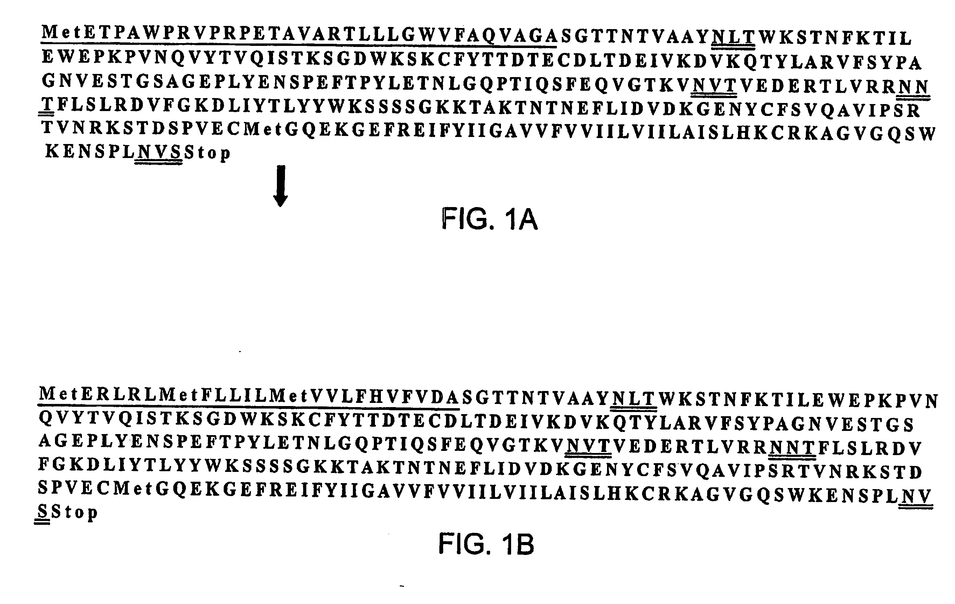

See also Figure 1A (showing the amino acid sequence of immature human tissue factor (SEQ ID NO.I)i.e., the mature sequence (Table **IA**) with the signal peptide (Table IB). References herein to an amino acid position of the human tissue factor protein refer to the mature protein shown above as Table IA unless otherwise specified.

By the phrase "recombinant" is meant that the techniques used for making the plant-derived tissue factor protein including those generally associated with making recombinant nucleic acid methods as well as plant culture and transformation (e.g., electroporation, lipofection). Generally, enzymatic reactions and purification steps are performed according to a manufacturer's specifications or in accordance with accepted protocols. The techniques and procedures are generally performed according to conventional methods in the art and various general references. See generally, Sambrook et al., Molecular Cloning: A Laboratory Manual, 2nd. edition (1989) Cold Spring Harbor Laboratory Press, Cold Spring Harbor, N.Y.

For more plant-specific recombinant methods, see Roger P. et al. (2000) Plant Mol. Biol. 42:819; Gartland K.M.A. and Davey M.R. (1995) Agrobacterium Protocols IN Methods in Molecular - 44, Humana Press, Totowa, N.J.; Hellens, R. et al. (2000) Trends in Plant Science, 5:10: 446; and Christou, P. (1996) Trends Plant Sci. 1: 423.

Also meant by the phrase "human tissue factor" including "rhTF" are. allelic variants of the mature human sequence shown in Table I (SEQ ID NO. as well as sequences that are at least about 70% identical thereto at the protein level, preferably at least about 80% identical, more preferably at least about 90% identical, and more preferably at least about 95%, about 99% or 100% identical. Methods for determining amino acid sequence identity are generally known in the field and include determinations made by using WU-BLAST software (Washington University BLAST) version 2.0. That program is reported to build on WU-BLAST version 1.4, which in turn is based on the public domain NCBI-BLAST version 1.4 (Altschul and Gish, (1996), Doolittle ed., Methods in Enzymology 266:460-480; Altschul et al., (1990), Journal of Molecular Biology 215:403-410; Gish and States, (1993), Nature Genetics 3:266-272; and Karlin and Altschul, (1993), Proc. Natl. Acad. Sci. USA 90:5873-5877). WU-BLAST version 2.0 executable programs for several UNIX platforms can be downloaded, for instance, from the Washington University BLAST website.

Examples of human tissue factor sequences that are less than 100% identical to the specified sequence are those having gaps (consecutive and/or nonconsecutive gaps of less than about 5 amino acids such as 1 or 2 amino acids) as well as those sequences featuring conservative amino acid substitutions (e.g., alanine substituted for leucine). Also envisioned are human tissue factor sequences having about 10 amino acid additions or less added to one or both of the N- or C- terminii.

By the phrase "rbTF" is meant recombinant human tissue factor.

An especially preferred human tissue factor protein sequence is shown (bound to a plant-derived signal peptide) in Figure 1B (SEQ ID NO. 2) at amino acid positions 1 to 263. See also Examples 1-3 below, for a preferred method of making that sequence. Also preferred is human tissue factor protein sequence from amino acid positions 1 to 243, fused to a plant-derived signal peptide.

In mammalian cells, TF is found as a transmembrane protein on the surface of mammalian cells that activates the extrinsic pathway for blood coagulation. It functions as a receptor that binds Factor VTIa. The TF-FVIIa complex functions to bind and activate Factor IX or X via limited proteolysis. The conversion of Factor IX or Factor X is considerably more efficient when the TF-FVIIa complex is associated with a membrane (Kalafatis, et al. (1997) Critical Reviews in Eukaryotic Gene Expression 7:241-280). In the case where TF protein is purified from a mammalian source or as a recombinant protein, (produced in a heterologous host such as *E. coli* and purified), the TF protein must be relipidated to provide an artificial membrane for optimal functioning. The purification of the TF protein is an expensive process and the relipidation is. expensive due to the cost of the artificial lipids used and the additional processing. A process for producing functionally active TF without the need to purify or relipidate the TF protein would be commercially important.

The targeting of the TF protein to the cell membrane is provided by a leader or signal sequence that is removed by proteolysis from the mature transmembrane protein. In the case of plants producing recombinant proteins, it has been shown that the mammalian signal sequence can function to target the protein to the membrane (Schillberg, et al. (1999) Transgenic Research 8:255-263), but plant derived signal sequences work better. Such plant signal sequences can include the signal sequence for the tomato subtilase (Janzik, et al. (2000) J. Biol. Chem. 275:5193-5199), the signal sequence for the rice alpha-amylase (McCormick, et al. (1999) PNAS 96:703-708), and the signal sequence for the Nicotiana calreticulin (Komamytsky, et al. (2000) Plant Physiology 124:927-933).

As discussed, the features a recombinant mammalian tissue factor that include an operatively linked and plant-derived signal peptide. Preferably, the signal sequence is derived from any suitable plant such as tomato, rice, tobacco, mustard, cottonmaize or alfalfa.

During the translocation of the TF to the membrane, the TF protein typically undergoes post translational modification, such as glycosylation. The full-length tissue factor protein has four sites for potential N-linked glycosylation and the 243 amino acid form of TF has three sites for potential N-linked glycosylation. The nature of the post-translational glycosylation by plants and mammalian cells are similar in that they contain N-acetyl glucosamine, fucose and mannose, although the nature of the linkages differ. But they differ in that plant glycosylation includes xylose which is not found in mammalian cells, and mammalian glycosylation can have terminal galactose and sialic acid residues not found in plant glycan structures (Ma, et al. (2003) Nature Reviews/Genetics 4:794-805).

Plant-specific glycans are known and have been reported. Generally, the glycans are added post-translationally to form a long-chain glycan structure. By "long-chain" is meant less then about 10 carbohydrate units (e.g., mannose, xylose, sialic acid, etc.) as a unbranched or preferably a branched glycan structure. More specifically, each of the glycans are added to one or more suitable glycosylation sites on a protein as the protein passes through the secretory. pathway. Most plant-expressed proteins including those disclosed herein will lack at least one of and preferably both of terminal galactose and sialic acid residues that are usually found in mammals. By "terminal" is meant that the carbohydrates would be present (in mammals) at the end of the long-chain glycan structure. However unlike mammals, the plant expressed proteins will include at least one of and preferably both of a α(1,3) fucose and a β(1,2) xylose group. See generally Warner, T.G. (2000) in Carbohydrates in Chemistry and Biol. (eds. Ernst, B. et al.) pp. 1043-1064 (Wiley, NY).

Accordingly, and in one embodiment, the invention provides a recombinant mammalian tissue factor protein that includes at least one plant-specific glycan, wherein the plant-specific glycan lacks at least one and preferably both of terminal galactose and terminal acetylneuraminic acid (NeuAc; sialic acid) group and includes at least one of and preferably both of a α(1,3) fucose and a β(1,2) xylose group. The plant-specific glycan includes preferably at least one of the α(1,3) fucose and a β(1,2) xylose group. In one embodiment, the α(1,3) fucose will be fused (covalently linked) to a N-acetylglucosamine (GlcNAc) group which GIcNAc group can, in one embodiment, be bound directly to a protein glycosylation site. Depending, for example, on the plant selected, the GlcNac group can be further bound to second GlcNac group as part of the long-chain glycan structure. The second GlcNac group can, in one embodiment, be bound to other carbohydrate groups of the glycan structure including mannose or GlcNac.

Optionally, the β(1,2) xylose group is covalently linked to a mannose group which group can be bound to one or more other GlcNac groups as described above, for instance. The mannose group can be further covalently linked to other mannose groups (usually two(2)) in a branched structure, which groups can be further bound to GlcNac. The plant-derived rhTF could also be produced by lettuce plants that have been genetically modified to alter the nature of the post-translational glycosylation.

An exemplary long-chain glycan has the following structure: 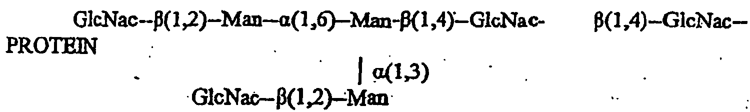 in which GlcNac is N-acetylglucosamine; and Man is mannose. See Ma., JK et al. Nature Reviews Vol. 4: 794 (2003) for additional information regarding illustrative long-chain glycan structures made by plants.

Also provided is a substantially purified preparation of the recombinant mammalian tissue factor disclosed herein. By the phrase "substantially pure" is meant molecules, either nucleic or amino acid sequences, that are removed from their natural environment, isolated or separated, and are at least 60% free, preferably 75% free and more preferably 90% free from other components with which they are naturally associated. Methods for making substantially pure proteins are disclosed herein and generally include centrifugation, chromatography, and related standard steps. An "isolated protein" is, therefore, a substantially purified protein.

Although, not needed the lettuce-derived tissue factor proteins disclosed herein can be "lipidated", for instance with at least one of, preferably broth, phosphatidylcholine and phosphatidylserine. Methods for lipidating tissue factor are known in the field and can be adapted as needed to lipidate the present proteins.

In this application, the TF protein is produced by lettuce and is associated with or bound to plant membranes. It is well known that "the biochemistry and cellular organization of fatty acid and glycerolipid synthesis in plant cells is quite different from the animal paradigm" (Miquel and Browse (1992) J Biochem 267:1502-1509). A comparison of the composition of plant cell versus animal cell membranes indicates that there are some similarities, but also numerous differences (see reviews on plant membranes - J.B. Mudd (1980) "Phospholipid Biosynthesis" pp.249-282 in The Biochemistry of Plants in P.K. Stumpf, ed. Academic Press, New York; mammalian membranes - H. Hauser & G. Poupart (1992) "Lipid Structure" pp. 3-71 in The Structure of Biological Membranes P. Yeagle, ed. CRC Press, Boca Raton, FL). Specifically, while both mammalian and plant membranes contain substantial amounts of phosphatidylcholine (PC) and phosphatidylethanolamine (PE), mammalian membranes contain higher proportions (on the order of 7-17%) of phospbatidylserine (PS), whereas plant membranes contain very little (1% or less). In addition, the fatty acids in plant membranes rarely have chain lengths greater than 18 carbon units, whereas the fatty acids in mammalian membranes can have longer chain lengths. The fatty acids in plant membranes tend to be more unsaturated with 18:3 (18 carbon chain length: 3 double bonds) predominating in plants, but seen only in trace amounts in mammalian membranes.

Given the differences in the membranes and glycosylation with mammalian cells compared to plants, it was interesting to find that in the invention of this application, the TF preparations in which the TF protein is in association with plant membranes were at least as active as TF protein purified from plants expressing TF and relipidated using PC (75%) and PS (25%) (van t' Veer, et al. (1997) J. Biol. Chem. 272:7983-7994). This was demonstrated using the TF preparations to stimulate clotting in the Prothrombin Time in vitro assay (shown in Example 5).

Described are transgenic plants that express tissue factor, preferably human tissue factor.

One or a combination of standard methods for expressing heterologous proteins in plants can be used to express the tissue factor protein as needed.

For instance, a large number of particular plant production systems have been developed. These include expressing protein on oil bodies (Rooijen et al., (1995) Plant Physiology 109:1353-61; Liu et al., (1997) Molecular Breeding 3:463-70), through rhizosecretion (Borisjuk et al., (1999) Nature Biotechnology 17:466-69), in seed (Hood et al., (1997) Molecular Breeding 3:291-306; Hood et al., (1999) In Chemicals via Higher Plant Bioengineering (ed. Shahidi et al.) Plenum Publishing Corp. pp. 127-148; Kusnadi et al., (1997) Biotechnology and Bioengineering 56:473-84; Kusnadi et al., (1998) Biotechnology and Bioengineering 60:44-52; Kusnadi et al., (1998) Biotechnology Progress 14:149-55; Witcher et al., (1998) Molecular Breeding 4:301-12), as epitopes on the surface of a virus (Verch et al., (1998) J. Immunological Methods 220:69-75; Brennan et al., (1999) J. Virology 73:930-38; Brennan et al., (1999) Microbiology 145:211-20), and by stable expression of proteins in potato tubers (Arakawa et al., (1997) Transgenic Research 6:403-13 ; Arakawa et al., (1998) Nature Biotechnology 16:292-97 ; Tacket et al., (1998) Nature Medicine 4:607-09). Recombinant proteins can also be targeted to the seeds, chloroplast or secreted to identify the location that gives the highest level of protein accumulation. Each of these could be adapted to express the tissue factor or fragment in a suitable plant host.

Additional general methods for expressing proteins in plants have been reported. See WO 03012035 to Bascomb, N. et al.; and WO002101006 to Hall, G. et al. These could be readily adapted to express the tissue factor protein or fragment in, for instance, *Arabadopsis* as well as a variety of other plants.

Further methods for expressing heterologous proteins in monocotyledenous and dicotyledenous plants have been reported. These include approaches that result in stable and constitutive expression of the protein of interest: 1) *Agrobacterium-mediated* gene transfer; 2) direct DNA uptake including methods for direct uptake DNA into protoplasts; 3) DNA uptake induced by brief electric shock of plant cells, 4) DNA injection into plant cells or tissues by particle bombardment, by the use of micropipette systems or by the direct incubation of DNA with germinating pollen; and 5) the use of plant virus as gene vectors. One or a combination of these methods can be used to create plants that express tissue factor and functional fragments thereof.

See e.g., Paszkowski, J., et al. (1989) in Cell Culture and Somatic Cell Genetics of Plants, Vol. 6, Molecular Biology of Plant Nuclear Genes eds. Schell, J., and Vasil, L. K., Academic Publishers, San Diego, Calif. p. 52-68; Klein, T. M., et al. in Progress in Plant Cellular and Molecular Biology, eds. Nijkamp, H. J. J., Van der Plas, J. H. W., and Van Aartrijk, J., (1988) Kluwer Academic Publishers, Dordrecht, p. 56-66; DeWet, J. M. J., et al. (1985) In Experimental Manipulation of Ovule Tissue, eds. Chapman, G. P. and Mantell, S. H. and Daniels, W. Longman, London, p. 197-209; Zhang, H. M. et al., (1988) Plant Cell Rep. 7:379-384; Frornn, M. E. et al., (1986) Nature 319:791-793; Hess, D. (1987) Int. Rev. Cyto. 107:367-395; Klein, T. M. et al., (1988) Bio/Technology 6:559-563; Neuhaus G. et al., (1987) Theor. Appl. Genet. 75:30-36; Neuhaus, G. and Spangenberg, G., (1990) Physiol. Plant. 79:213-217; Ohta, Y. (1986) Proc. Natl. Acad. Sci. USA 83:715-719; and US Published Patent Application 2003/0138456.

### Agrobacterium-mediated Gene Transfer

Gene transfer by means of engineered *Agrobacterium* strains has become routine for most dicotyledonous plants and for some monocotyledonous plants. See e.g., Fraley et al. (1983) Proc. Natl. Acad. Sci. USA 80:4803; Watson et al. (1985) EMBO J 4:277; Horsch et al. (1985) Science 227:1229; Hernalsteens et al. (1984) EMBO J 3:3039; Comai et al. (1984) Nature (London) 317:741; Shah et al. (1986) Science 233:478; Bytebier et al. (1987) Pro. Natl. Acad. Sci. USA 84:5345; Schafew et al. (1987) Nature 327:529; Potrykus, I. (1990) Biotechnol 8:535; Grimsley et al. (1987) Nature 325:177; Gould et al (1991) Plant Physiol 95:426; Ishida et al. (1996) Nature Biotechnology 14:745; and U.S. Pat. No. 5,591,616.

An appropriate Agrobacterium strain utilized in the methods of the invention is modified to contain a gene or genes of interest, or a nucleic acid to be expressed in the transformed cells. The nucleic acid to be transferred is incorporated into the T-region and is flanked by at least one T-DNA border sequence. A variety of *Agrobacterium* species are known particularly for dicotyledon transformation. Such *Agrobacterium* can be used in the methods of the invention. See, for example, Hooykaas, P. J. (1989) Plant Mol. Biol. 13:327; Smith et al. (1995) Crop Science 35:301; Chilton, M. O. (1993) Proc. Natl. Acad. Sci. USA 90:3119; Mollony et al. N:Monograph Theor Appl Genet NY, Springer verlag 19:148, 1993; and Ishida et al. (1996) Nature Biotechnol. 14:745; Komari, T. et al. (1996) The Plant Journal 10:165.

In the Ti plasmid, the T-region is distinct from the *vir* region whose functions are responsible for transfer and integration. Binary vector systems have been developed where the manipulated disarmed T-DNA carrying foreign DNA and the *vir* functions are present on separate plasmids. In this manner, a modified T-DNA region comprising foreign DNA (the nucleic acid to be transferred) is constructed in a small plasmid which replicates in *E. coli.* This plasmid is transferred conjugatively in a tri-parental mating or via transformation into A. *tumefaciens* which contains a compatible plasmid-carrying virulence gene. The *vir* functions are supplied in *trans* to transfer the T-DNA into the plant genome. Such binary vectors are useful. Disclosure relating to making and using a variety of Ti vectors has been published. See USP Nos. 5,276,268; 5,283,184; and 5,286,635 for examples of suitable Ti vectors.

Further vectors for use include those "super-binary" disclosed in U.S. Pat. No. 5,591,616 and EPA 0604662A1, for instance. See also Hood et al. (1984) Biotechnol. 2:702-709; Hood et al. (1986) J. Bacteriol. 168:1283-1290; Komari et al. (1986) J. Bacteriol. 166:88-94; Jin et al. (1987) J. Bacteriol. 169:4417-4425; Komari T. (1989) Plant Science 60:223-229; ATCC Accession No. 37394).

Other appropriate vectors that can be used such as those described by deFramond, A. et al., (1983) Bio/Technology 1:263; An, G. et al., (1985) EMBO J. 4:277; and Rothstein, S. J. et al., (1987) Gene 53:153.

Other sequences for facilitating site-specific genome integration and/or controlled excision and/or reinsertion into the genome may also be provided. For example, the Cre/lox system can be used to obtain targeted integration of an *Agrobacterium* T-DNA at a lox site in the genome of *Arabidopsis.* Site-specific recombinants, and not random events, are preferentially selected by activation of a silent lox-neomycin phosphotransferase (nptII) target gene. Cre recombinase can be provided transiently by using a co-transformation approach. See, e.g., as described in Vergunst, et al., (1998) Plant Mol Biol 38(3):393-406.

A vector suitable for chloroplast transformation can be used in accord with the invention as well. Chloroplasts are prokaryotic compartments inside eukaryotic cells. Since the transcriptional and translational machinery of the chloroplast is similar to *E*. *coli* (Brixey et al., (1997) Biotechnology Letters 19:395-400), it is possible to express prokaryotic genes at very high levels in plant chloroplasts than in the nucleus (Daniell, et al. (2002) Trends in Plant Science 7:84-91; Tregoning, et al. Nucleic Acid Res. 31:1174-1179). In addition, plant cells contain up to 50,000 copies of the circular plastid genome (Bendich (1987) Bioessays 6:279-282) which may amplify a recombinant gene like a plasmid, enhancing levels of expression. In some invention embodiments, chloroplast expression may be a hundred-fold higher than nuclear expression in transgenic plants (Daniell, WO 99/10513).

Additionally suitable vectors for use with the invention are replicable, preferably in a microbial host such as a bacterium (e.g., *E. coli*) and include the following components: (a) a first promoter operable in a plant; (b) an expressible nucleic acid sequence encoding a mammalian tissue factor protein, wherein the nucleic acid sequence is operatively associated with the promoter; and (c) a termination sequence operatively associated with the nucleic acid sequence.

The term "promoter" refers to the nucleotide sequences at the 5' end of a structural gene which directs the initiation of transcription. Generally, promoter sequences are necessary, but not always sufficient, to drive the expression of a downstream gene. In the construction of heterologous promoter/structural gene combinations, the structural gene is placed under the regulatory control of a promoter such that the expression of the gene is controlled by promoter sequences. The promoter is positioned preferentially upstream to the structural gene and at a distance from the transcription start site that approximates the distance between the promoter and the gene it controls in its natural setting. As is known in the art, some variation in this distance can be tolerated without loss of promoter function. As used herein, the term "operatively linked" means that a promoter is connected to a coding region in such a way that the transcription of that coding region is controlled and regulated by that promoter. Means for operatively linking a promoter to a coding region are well known in the art.

### Illustrative Plant Promoters

The gene constructs used may include all of the genetic material and such things as promoters, IRES elements, etc. These expression cassettes can either require some external stimuli to induce expression, such as the addition of a particular nutrient or agent, change in temperature, etc. or can be designed to express an encoded protein immediately and/or spontaneously during growth.

Thus, the expression of a gene encoding a desired protein may be controlled by constitutive or regulated promoters. Regulated promoters may be tissue-specific, developmentally regulated or otherwise inducible or repressible, provided that they are functional in the plant cell. Regulation may be based on temporal, spatial or developmental cues, environmentally signaled, or controllable by means of chemical inducers or repressors and such agents may be of natural or synthetic origin and the promoters may be of natural origin or engineered. Promoters also can be chimeric, i.e., derived using sequence elements from two or more different natural or synthetic promoters.

Preferably, a promoter used in the construct yields a high expression level of the gene, allowing for accumulation of the protein to be at least about 0.1-1%, at least about 1-5%, and more preferably, at least about 5% of total soluble protein, and/or yields at least about 0.1 %, preferably at least about 0.5%, and most preferably, at least about 1%, of the total intercellular fluid (ICF) extractable protein.

The promoter should preferentially allow expression in all of the plant tissues, but most preferably, in all of the leaf, stem and root tissue. Additionally, or alternatively, the promoter allows expression in floral and/or seed tissue. In the present invention, the *Arabidopsis Actin* 2 promoter, the (OCS)3MAS promoter and various forms thereof, the CaMV 35S, and figwort mosaic virus 34S promoter are preferred. However, other constitutive promoters can be used. For example, the ubiquitin promoter has been cloned from several species for use in transgenic plants (e.g., sunflower (Binet et al., (1991) Plant Science 79:87-94; and maize (Christensen et al., (1989) Plant Molec. Biol. 12:619-632). Further useful promoters are the U2 and U5 snRNA promoters from maize (Brown et al., (1989) Nucleic Acids Res. 17:8991) and the promoter from alcohol dehydrogenase (Dennis et al., (1984) Nucleic Acids Res. 12:3983).

In another aspect, a regulated promoter is operably linked to the gene. Regulated promoters include, but are not limited to, promoters regulated by external influences (such as by application of an external agent, e.g., such as chemical, light, temperature, and the like), or promoters regulated by internal cues, such as regulated developmental changes in the plant. Regulated promoters are useful to induce high-level expression of a desired gene specifically at, or near, the time of harvest. This may be particularly useful in cases where the desired protein limits or otherwise constrains growth of the plant, or is in some manner, unstable.

Plant promoters which control the expression of transgenes in different plant tissues by methods are known to those skilled in the art (Gasser & Fraley, (1989) Science 244:1293-99). The cauliflower mosaic virus *35S* promoter (CaMV) and enhanced derivatives of CaMv promoter (Odell et al., (1985) Nature, 3(13):810), actin promoter (McElroy et al., (1990) Plant Cell 2:163-71), AdhI promoter (Fromm et al., (1990) Bio/Technology 8:833-39, Kyozuka et al., (1991) Mol. Gen. Genet 228:40-48), ubiquitin promoters, the Figwort mosaic virus promoter, mannopine synthase promoter, nopaline synthase promoter and octopine synthase promoter and derivatives thereof are considered constitutive promoters. Regulated promoters are described as light inducible (e.g., small subunit of ribulose biphosphatecarboxylase promoters), heat shock promoters, nitrate and other chemically inducible promoters (see, for example, U.S. Patents 5,364,780; 5,364,780; and 5,777,200).

Tissue specific promoters are used when there is reason to express a protein in a particular part of the plant Leaf specific promoters may include the C4PPDK promoter preceded by the 35*S* enhancer (Sheen, (1993) EMBO, 12:3497-505) or any other promoter that is specific for expression in the leaf. For expressing proteins in seed, the napin gene promoter (U.S. Patents 5,420,034 and 5,608,152), the acetyl-CoA carboxylase promoter (U.S. Patent 5,420,034 and 5,608,152), 2S albumin promoter, seed storage protein promoter, phaseolin promoter (Slightom et. al., (1983) Proc. Natl. Acad Sci. USA 80:1897-1901), oleosin promoter (Plant et al., (1994) Plant Mol. Bio. 25:193-205; Rowley et. al., 1997, Biochim. Biophys. Acta. 1345:1-4; U.S. Patent 5,650,554; PCT WO 93/20216), zein promoter, glutelin promoter, starch synthase promoter, and starch branching enzyme promoter are all useful.

Plant promoters can be selected to be inducible or constitutive as needed to achieve an intended invention result.

Generally, any plant expressible genetic construct is suitable for use in the methods of the invention. Particular promoters may be selected in consideration of the type of recombinant protein being expressed.

Other regulatory elements such as enhancer sequences also may be provided. For example, in one aspect, expression cassettes that contain multimerized transcriptional enhancers from the cauliflower mosaic virus (CaMV) 35S gene are used. See, e.g., Weigel, et al. (2000) Plant Physiol 122(4):1003-13.

If desired, suitable vectors for use with the invention can further include a sequence that suppresses gene silencing in plants. Such sequences are generally derived from plant viruses and have been disclosed. See Anandalakshimi, R. et al. (1998) PNAS (USA) 95: 13079.

### Illustrative Termination Sequences

In the vector described in this invention the nopaline synthase gene (Nos) terminator is used. Other terminators include e.g., octopine synthase gene (OCS) terminator (Halfter, et al. (1992) Mol Gen Genet 231:186-193) or the cruciferin A (cruA) terminator (Rodin, et al. (1992) Plant Mol Bio 20:559-563) can be used.

### Selectable Markers and/or Reporter Genes

Selectable markers, such as antibiotic (e.g., kanamycin (*nptII*) and hygromycin (*hpt*)) resistance, herbicide (glufosinate, imidazlinone, glyphosate, AHAS, EPSPS) resistance or physiological markers (visible or biochemical) are used to select cells transformed with the nucleic acid construct. Non-transgenic cells (i.e., non-trans formants) on the other hand, are either killed or preferentially do not grow under the selective conditions. In one aspect, a selectable marker gene is a gene which encodes a protein providing resistance or physiological markers. However, in another aspect, a selectable marker gene is a gene encoding an antisense nucleic acid.

Reporter genes may be included in the construct or they may be contained in the vector that ultimately transports the construct into the plant cell. As used herein, a "reporter gene" is any gene which can provide a cell in which it is expressed with an observable or measurable phenotype.

Expression of reporter genes yields a detectable result, e.g., a visual colorimetric, fluorescent, luminescent or biochemically assayable product; a selectable marker, allowing for selection of transformants based on physiology and growth differential; or display a visual physiologic or biochemical trait. Commonly used reporter genes include lacZ (β-galactosidase), GUS (β-glucuronidase), GFP (green fluorescent protein and mutated or modified forms thereof), luciferase, or CAT (chloramphenicol acetyltransferase), which are easily visualized or assayable. Such genes may be used in combination or instead of selectable markers to enable one to easily pick out clones of interest. In one aspect, a selectable marker gene is a gene encoding a protein product.

Selectable markers can also include molecules that facilitate isolation of cells which express the markers. For example, a selectable marker can encode an antigen which can be recognized by an antibody and used to isolate a transformed cell by affinity-based purification techniques or by flow cytometry. Reporter genes also may comprise sequences which are detected by virtue of being foreign to a plant cell (e.g., detectable by PCR, for example). In this aspect , the reporter gene need not express a protein or cause a visible change in phenotype.

In a more particular aspects of the previously mentioned replicable vector, the vector further includes d) a second promoter operable in the plant, (e) a selectable marker gene operatively associated with the second promoter, (f) an *Agrobacterium* TR sequence, and (g) an *Agrobacterium* TL sequence in which the expressible nucleic acid sequence encoding the mammalian tissue factor protein or fragment further encodes a plant specific signal peptide operatively linked to the tissue factor protein. The expressible nucleic acid sequence encoding the mammalian tissue factor is typically at least about 70% identical to the mature human TF sequence shown in Table I as SEQ ID NO. 4 preferably at least about 80%, about 90%, or at least about 99% or 100% identical to that sequence. Alternatively, or in addition, the encoded mammalian tissue factor or fragment exhibits at least about 70% of the prothrombin time of the mature human TF sequence shown in Table I as SEQ ID NO. 4 as monitored in a standard prothrombin time (PT) assay.

By the phrase "standard prothrombin time" assay is meant the assay generally described by Miletich, JP (1995) in Prothrombin Time (Chapter L33) in Williams Hematology, 5th Ed. (Beutler, E. et al. Eds.) McGraw-Hill, Inc., Heath Professions Div., New York, pages L82-L84,

Generally, the standard prothrombin time assay includes the steps of diluting a tissue factor sample (e.g., crude plant extract that includes TF) in an acceptable buffer to make a serial dilution (about 0.2 mL total). A preferred buffer is PT buffer (see Example 5). The assay can be initiated by adding about 0.1. mL of blood plasma, preferably pre-incubated with the buffer. Coagulation' can be monitored by one or a combination of different strategies such as use of an automated coagulation timer. Examples of such devices are generally available from commercial vendors.

A specific example of the standard prothrombin time assay is provided in Example 5.

Also envisioned are transgenic lettuce plant progeny obtained from the cutting, slip, cultivar, or seed of the foregoing transgenic lettuce plants in which the progeny plant also includes the expressible nucleic acid sequence encoding the mammalian tissue factor protein.

As discussed, in the Example section, *L. sativa* (lettuce) is the plant of choice for expressing the mammalian tissue factor or fragment. In this aspect the protein can be expressed stably or transiently as needed to achieve an intended result. Lettuce is the plant of choice particularly in aspect in which large-scale production of the protein is desirable. The fleshy, large leave and relatively simple growth requirements, and other characteristics will serve this objective. As discussed below in more detail, it has also been found that lettuce is an especially amenable for the transient expression of tissue factor. If desired however, lettuce can be used to stably express the tissue factor using, for instance, suitable transformation methods described herein.

Described is a transgenic lettuce (*L*. *sativa*) that includes transformed plant cells in which the cells comprise at least one and preferably all of the following:
(a) a first promoter operable in a plant,
(b) an expressible nucleic acid sequence encoding a mammalian tissue factor protein, wherein the nucleic acid sequence is operatively associated with the promoter; and
(c) a termination sequence operatively associated with the nucleic acid sequence.
(d) a second promoter operable in the plant,
(e) a selectable marker gene operatively associated with the second promoter,
(f) an *Agrobacterium* TR sequence, and
(g) an *Agrobacterium* TL sequence.

Preferably, the expressible nucleic acid sequence encoding the mammalian tissue factor protein further encodes a plant specific signal peptide operatively linked to the tissue factor protein or fragment. In a more particular aspect of the transgenic lettuce: i) the first promoter is inducible or constitutive; ii) the expressible nucleic acid sequence encodes the mature human tissue factor sequence shown in Table I as SEQ ID NO. 4 a functional fragment thereof or a sequence at least about 70% identical to the mature human tissue factor sequence shown in Table I as SEQ ID NO. 4 (preferably at least about 80% or 90%) and exhibiting at least about 70% of the prothrombin time (PT) of the tissue factor protein shown in Table I as the mature human tissue factor protein (preferably at least about 80% or 90%)as determined by the standard PT assay; iii) the termination sequence is Nos-3'; iv) the second promoter is a 35S promoter; and v) the selectable marker gene is NPTII.

More preferred expressible nucleic acid sequences for use encode human tissue factor sequence with about the same activity in the PT assay as the mature human tissue factor sequence exemplified in Table I as SEQ ID NO. 4

### Transformation of Lettuce

For the use of lettuce nearly any of the transformation methods provided herein can be used to produce transgenic plants that express the tissue factor. Such expression can be stable or transient as needed. One or a combination of the methods described in WO 02101006 can be used including those relating to vacuum-infiltration and incubation of *Agrobacterium-*treated plant tissue. A specific protocol for transforming lettuce heads transiently is provided in the Examples.

### Crude Plant Extracts That Include TF

As discussed, it is an object to provide crude extracts of the transgenic plants disclosed herein which extracts will include mammalian tissue factor, preferably human tissue factor.

As also discussed, it has been surprisingly found that lettuce can express tissue factor that is biologically active. Thus it is a specific objective to provide such active tissue factor in crude form, e.g., in medical, veterinary or combat settings where functioning tissue factor is needed quickly and/or in large amounts regardless of purity.

By the phrase "crude extract" is meant an extract or lysate of plant tissue (leaves, stalks, flowers, roots, tubers, bulbs, fruits, seeds, etc) that is prepared by contacting the plant tissue with a pharmaceutically acceptable solution (water, phosphate-buffered saline (PBS), etc.), substantially disrupting the tissue (e.g., by use of a morter and pestle, french press, blender and the like) in the presence of between from about 1 to about 10 volumes of solution, and then removing debris by filtration or gentle centrifugation, typically about 1,000 x g for less then about 30 minutes, preferably between from about 5 to about 20 minutes. Of course, the precise amount of solution will add will depend on recognized parameters such as the plant selected and the concentration of tissue factor desired in the extract. The debris is removed and the remaining solution kept as the "crude extract". In most embodiments, one or a combination of pharmaceutically acceptable buffers are preferred (such as PBS) to help preserve tissue factor integrity and maximize potential therapeutic use. A variety of pharmaceutically acceptable solutions have been disclosed. See Remington's Pharmaceutical Sciences, (Mack Publishing Co., Easton PA, (1980)).

As mentioned, the crude extract as disclosed herein can provide mammalian tissue factor at relatively low cost and without time consuming relipidation. Crude extract is relatively stable and may be stored at low temperature (below freezing, e.g., -70C) until needed.

Typical crude extracts will have between about 1 ng/mL to 1 µg/mL of the mammalian tissue factor protein or functional fragment, or preferably between from about 1 µg/mL to 100 µg/mL.

### Tissue Factor Isolation From Crude Extract .

Although the mammalian tissue factor in the crude extract is biologically active i.e., it shows good activity in the standard PT assay without any relipidation, it will sometimes be useful to make a more purified tissue factor preparation. In some instances, a substantially pure preparation will be helpful.

In these aspects and after cultivation, the transgenic plant is harvested to recover the produced multi-subunit protein or processed protein (and/or other proteins produced by structural genes according to the invention). This harvesting step may comprise harvesting the entire plant, or only the leaves, or roots or cells of the plant. This step may either kill the plant or, if only the portion of the transgenic plant is harvested, may allow the remainder of the plant to continue to grow.

After harvesting, protein isolation may be performed using methods routine in the art. For example, at least a portion of the plant may be homogenized, and the protein extracted and further purified. Extraction may comprise soaking or immersing the homogenate in a suitable solvent. As discussed above, proteins may also be isolated from interstitial fluids of plants, for example, by vacuum infiltration methods, as described in U.S. Patent No. 6,284,875. Purification methods include, but are not limited to, immuno-affinity purification and purification procedures based on the specific size of a protein/protein complex, electrophoretic mobility, biological activity, and/or net charge of the multimeric protein to be isolated. Acceptable purification methods thus include at least one of chromatography, centrifugation, and immunoprecipitation (see USP 5,986,065 and 6,555,319 for illustrative anti-tissue factor antibodies).

In a more particular method, the crude extract is centrifuged at about 20,000 X g followed by gentle filtering through a 1 micron filter to yield a supernatant. That supernatant is subjected to chromatography (preferably Q Sepharose Fast Flow Resin). The flow through was subjected to affinity chromatography (preferably 147 affinity) followed by washing with an acceptable wash buffer. Flow through can be stored at low temperature directly or subjected to standard buffer exchange procedures. See the Examples below.

As mentioned, it is another object to provide coagulant compostions that include lettuce-derived mammalian tissue factor.

Such compositions can include, for instance, the crude extract. Alternatively, and when more purified TF is needed, the coagulants and coagulant compositions can include a substantially pure preparation of the tissue factor.

### Coagulant Formulations

The coagulant compositions can be employed, either alone or in combination with one or more other therapeutic agents or procedures that help minimize blood flow. Such agents include other blood factors (e.g., tissue factor fragments) or even solid supports such as cotton guaze. Illustrative techniques include applying pressure to the wound, cauterization or the use of sutures. In this regard, a preferred coagulant composition will include one or more conventional excipients, i.e., pharmaceutically acceptable organic or inorganic carrier substances suitable for parenteral, enteral or intranasal application which do not deleteriously react with the active compounds and are not deleterious to the recipient thereof.

Suitable pharmaceutically acceptable carriers include but are not limited to water, salt solutions, alcohol, vegetable oils, polyethylene glycols, gelatin, lactose, amylose, magnesium stearate, talc, silicic acid, viscous paraffin, perfume oil, fatty acid monoglycerides and diglycerides, petroethral fatty acid esters, hydroxymethyl-cellulose, polyvinylpyrrolidone, *etc.* The pharmaceutical preparations can be sterilized and if desired mixed with auxiliary agents, e.g., lubricants, preservatives, stabilizers, wetting agents, emulsifiers, salts for influencing osmotic pressure, buffers, colorings, flavorings and/or aromatic substances and the like which do not deleteriously react with the active compounds (typically the tissue factor). See *Remington's Pharmaceutical Sciences, infra.*

Typical amounts of crude extract and/or more purified tissue factor to use in a particular coagulant compositions will vary according to understood parameters such as intended use of the composition, the amount of blood flow anticipated, the particular tissue factor sequence selected, the rapidity of blood flow cessation needed etc. However in most instances, the amount of tissue factor or functional fragment in the compositions (expressed as w/w) will be between from about nanogram to about 5000 micrograms per milliliter of blood, for instance about 100 nanograms to about 50 micrograms.

### Treatment Methods And Other Uses

The coagulant compositions can be used in a wide variety of setting including diagnostic assays (PT, aPTT and other standard blood clotting tests). In this aspect, the coagulant composition can be used in place of relipidated TF.

However, often preferred is the coagulant composition for use to prevent or treat blood loss in a subject. In this instance, the use will include contacting the subject with a therapeutically effective amount of the anticoagulant disclosed herein. Alternatively, the use can be used with any of the crude extracts provided herein as well as the substantially pure mammalian. tissue factor.

In addition to its role in blood coagulation, TF plays a role in angiogenesis. This was discovered when it was found that mice in which TF had been genetically knocked out were unable to develop beyond embryonic day 9-10 due to the failure to develop blood vessels (Carmeliet, et al (1996) Nature 383:73-75; Bugge, et al. (1996) PNAS 93:6258-6263; Toomey, et al. (1996) Blood 88:1583-1587). Further studies have demonstrated that activation of coagulation proteases can lead to activation of the protease activated receptors leading to increased production of vascular endothelial growth factors which stimulate angiogenesis (Richard, et al. (2002) Oncogene 20:1556.1562; Milia, et al. (2002) Circ. Res. 91:346-352). In addition, the over-expression of TF on tumor cells promotes tumor growth, vascularization and metastasis (Mueller, et al. (1992) PNAS 89:11832-11836).

The roles of TF in promoting blood coagulation, angiogenesis and vascular development/remodeling are well documented. These roles are clearly important aspects of wound repair and healing. Several reports have indicated that TF promotes wound repair and healing using TF isolated from mammalian sources or produced by recombinant means in hosts other than plants (Nakagawa, et al. (1998) Seminars in Thromb. & Hemostasis 24:207-210; Philippart, et al. (2003) The Internatl J of Oral & Maxillofacial Impllants 3:411-416). To further study the effects on coagulation and wound healing in vivo, an animal model was used in which partial thickness wounds were made on the back of a pig (Eaglstein and Mertz (1978) J Invest. Dermatology 71:382-384; Alvarez, Mertz & Eaglstein (1983) J. Surgical Res. 35:142,148). Using this model, it was shown that the plant-derived TF effectively stopped bleeding from the wounds faster than controls and at least comparably to purified TF protein that had been relipidated (shown in an Example).

In addition, the invention provides the TF for use in promoting wound healing in a subject. In one embodiment, the: use includes contacting the subject with a therapeutically effective amount of any of the coagulant compositions disclosed herein.

Further uses in accord with the invention include promoting angiogenesis in a subject. In one embodiment, the use includes contacting the subject with a therapeutically effective amount of any of the anticoagulant compositions disclosed herein.

Further invention uses include promoting vascular remodeling in a subject. In one embodiment, the use includes contacting the subject with a therapeutically effective amount of any of the anticoagulant compositions disclosed herein.

The coagulant compositions can be used in the methods in nearly any acceptable form such as pouring or spaying the coagulant composition on or near a wound.

The invention also provides an absorbent (or adsorbent) that includes at least one of the coagulant compostions disclosed herein. Preferably, the absorbent is a medically acceptable support such as guaze (preferably sterile and including cotton or a cotton blend). Nearly any of the coagulant compositions can be administered to the absorbent e.g., as a dry formulation or in wet or moist form.

Also provided is an adhesive support that includes the absorbent. Preferably, the adhesive support is sterile. If desired, the adhesive support and absorbent can be placed or sealed into a wrapper container that is preferably impervious to water and air.

In one embodiment, this invention relates to the production of active recombinant human tissue factor (rhTF) in lettuce. It features an efficient and inexpensive way of production of biomass possessing rhTF, which can be used directly for bleeding cessation and wound healing. No purification or lipidation are required. A binary vector was constructed with the gene coding for full-length (263 aa) rhTF, which can be used both for transient expression and for stable transformation in plants, plant tissues or plant cells. In one example, the rhTF was produced transiently in lettuce. Two samples of this protein were prepared and tested. One sample of plant-derived rhTF was purified and lipidated (plant purified TF). A second sample was a fraction of a crude extract that had not been purified or lipidated artificially. The fraction was made by simply homogenizing leaf biomass with an equal volume of PBS which was then centrifuged for 5 min at 1,000 x g and 4°C. Supernatant was used as the crude extract. Both samples were tested for blood coagulation activity using prothrombin time assay. It was found that both samples possessed a high coagulation activity close to human TF. Animal study on pig model also demonstrated notable coagulation activity. Thus, using plant expression system one can produce inexpensive and highly active rhTF, which could be used both for bleeding cessation and wound healing with humans and other mammals.

In one aspect one can use cells of *Agrobacterium tumefaciens* bearing a binary vector with the gene for rhTF. This vector can be used both for transient expression and for stable transformation of lettuce, plant tissues or plant cells. In this vector, the eukaryotic segment comprises: TL (the left terminal repeat of the tumor-inducing region from the *Agrobacterium* TI plasmid), a promoter (inducible or constitutive), apoplast targeted signal peptide, gene of rhTF, termination transcription signal, a 35S promoter directing expression of NPTII gene, termination transcription signal, and TR (the right terminal repeat of the tumor-inducing region from the *Agrobacterium* TI plasmid).

In another aspect rhTF gene will be expressed transiently or in stable transgenic plants.

In another aspect, preparations comprising the rhTF protein can be obtained which are active without any purification procedure.

In another aspect, the rhTF fractions can be formulated in a liquid form, a lyophilized form for reconstitution or as a dry powder aerosolized application, as creams or other topical formulations, or as a powdered leaf biomass.

In another aspect, such formulations can be used for bleeding cessation and wound healing in humans or other mammals.

As discussed, the disclosure features a novel technology of production of rhTF and its use for prevention of intensive bleeding at wounded people or mammalian animals as well as for acceleration of the wound healing. It allows produce unlimited quantities of inexpensive source of rhTF which can be applied for wound treatments.

Further provided is a composition comprising soluble, functional human tissue factor produced by and obtained from transgenic lettuce is disclosed. Methods are also disclosed for the expression of genes for human tissue factor in transgenic plants, plant tissue or plant cells. More specifically, active tissue factor preparations comprising fractions of crude extracts from plant material containing the tissue factor protein can be made, as well as preparations in which the tissue factor is purified and relipidated. Furthermore, the fractions from crude extracts of plant material expressing tissue factor are active without requiring the introduction of exogenous lipids. Such preparations are active, as demonstrated by triggering coagulation *in vitro* and halting bleeding *in vivo.* Also disclosed are such preparations for use for promoting blood coagulation, minimizing blood loss and promoting wound closure and healing.

The following Examples are intended to illustrate the present invention.

### EXAMPLE 1: Preparation of Agrobacterium Vector Encoding Tissue Factor

This example describes the method of producing recombinant human tissue factor (rhTF) in lettuce. The method involves cloning the DNA sequence coding for tissue factor into a plasmid DNA vector that is capable of replication in *Escherichia coli* and *Agrobacterium tumifaciens.* This recombinant DNA molecule can be introduced into plants, plant tissues or cells, such that the TF gene is expressed.

### Binary vector construction

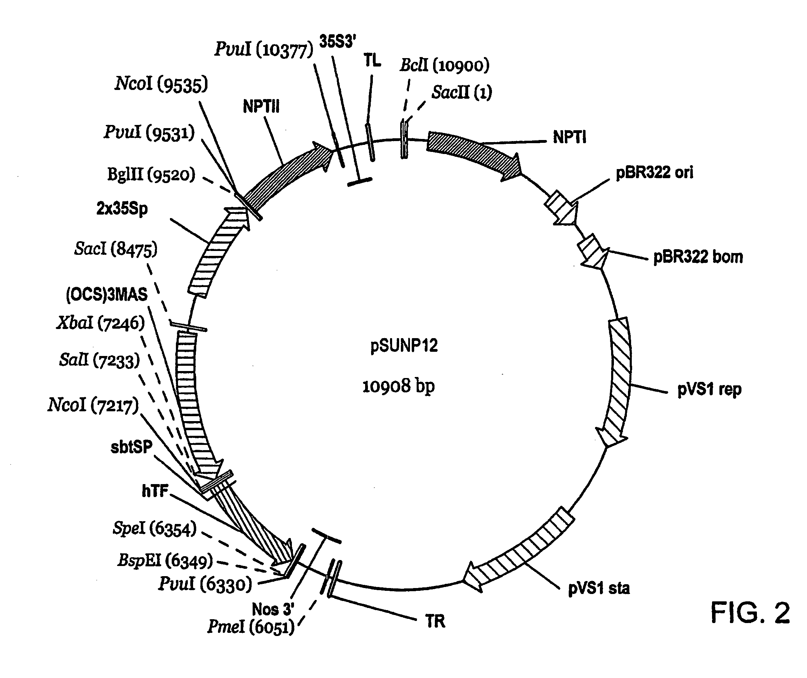

The binary vector was constructed so as to have a gene encoding full-length (263 aa) rhTF (Fig. 1), which can be used both for transient expression and for stable transformation. In Fig. 2, the map of one binary vector is shown comprising: TR, Nos-3' terminator (reverse orientation), gene of rhTF (reverse orientation) with tomato subtilase apoplast-targeting signal peptide (Janzik *et al.,* 2000) (reverse orientation), 2,4-D inducible (OCS)3Mas promoter (Gelvin et al., US Pat. #5,955,646) (reverse orientation), 35S promoter, NPTII gene, 35S-3' terminator, and TL.

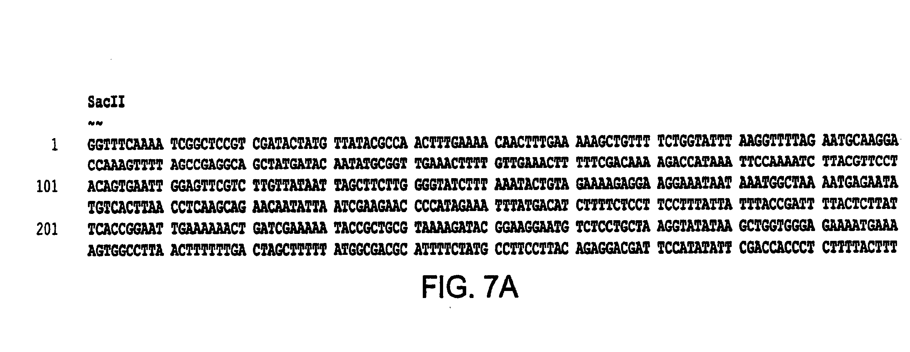

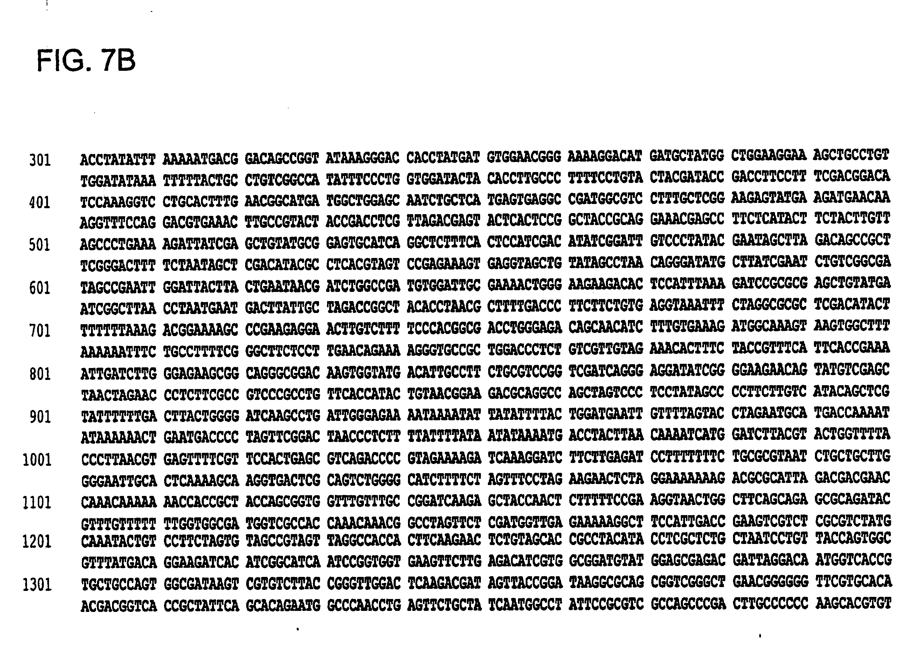

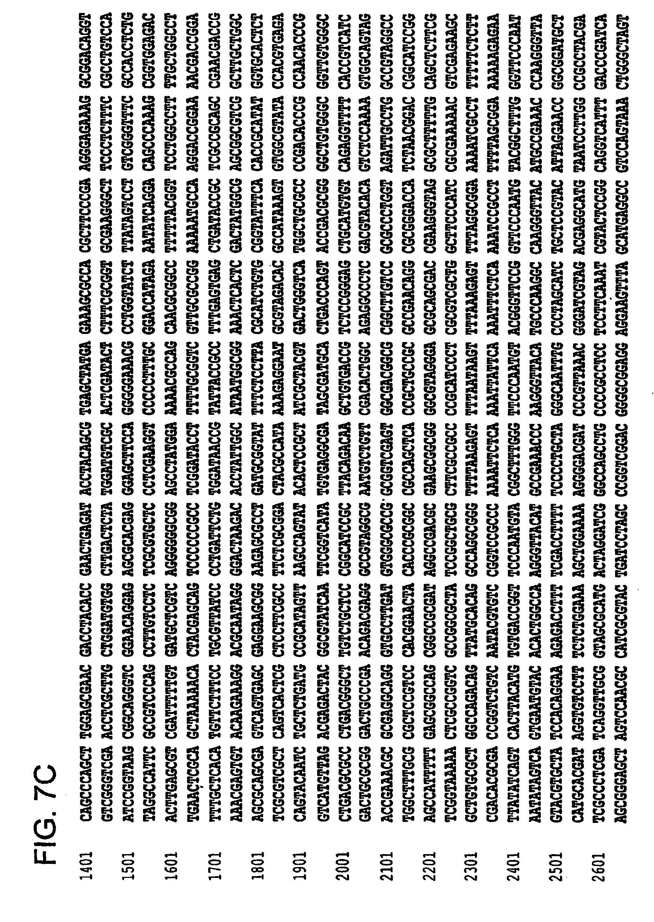

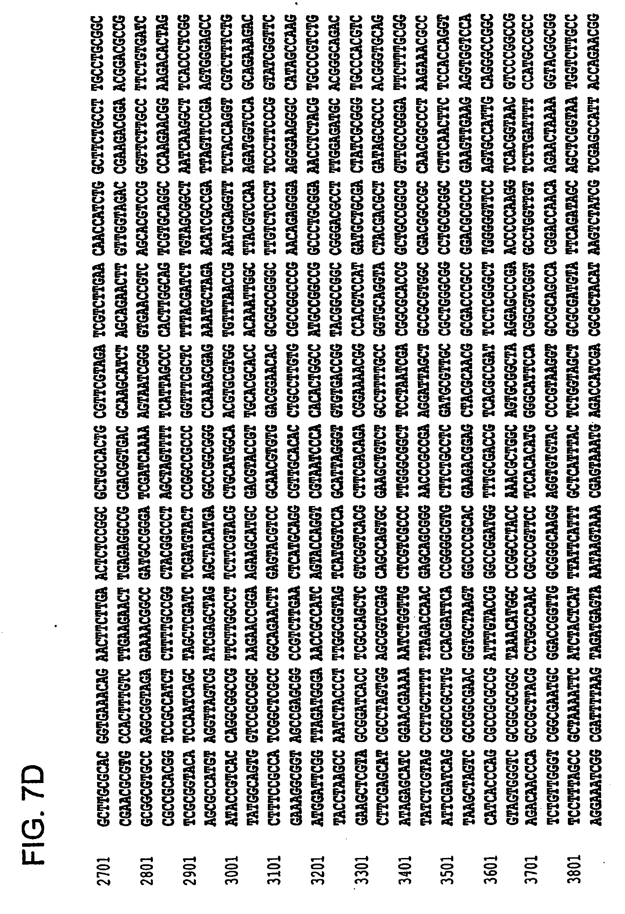

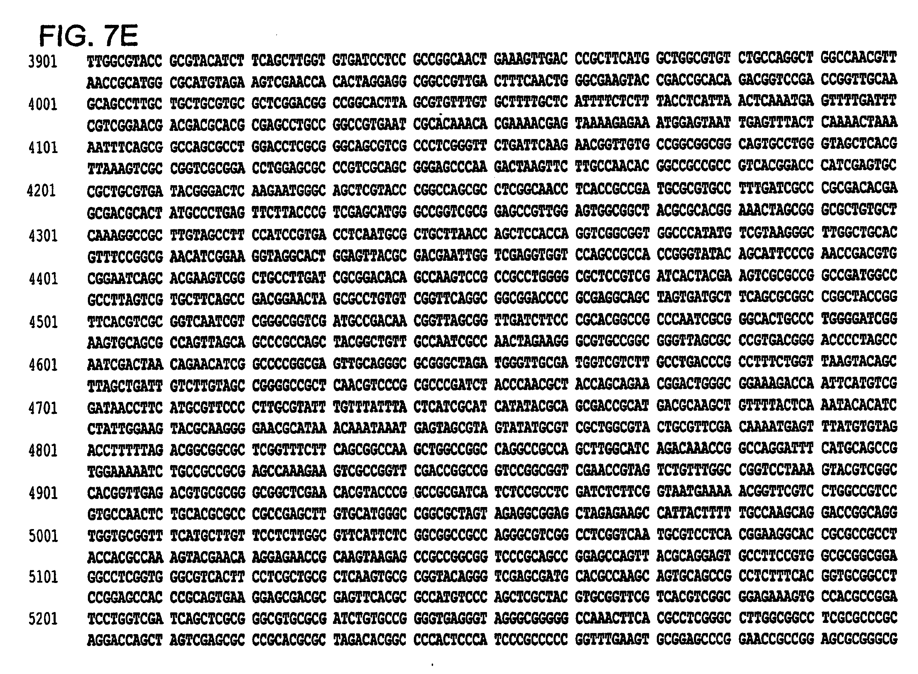

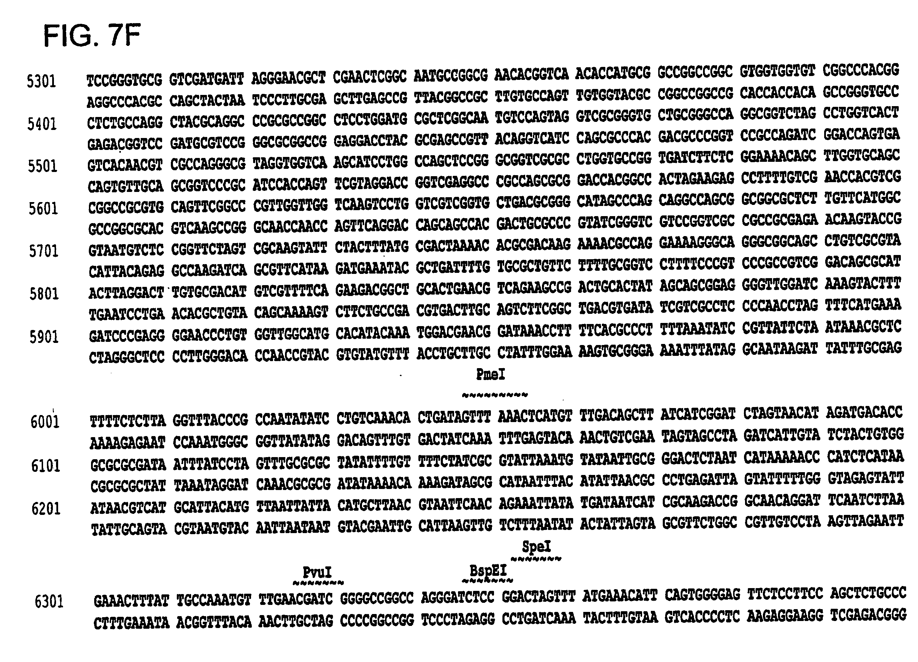

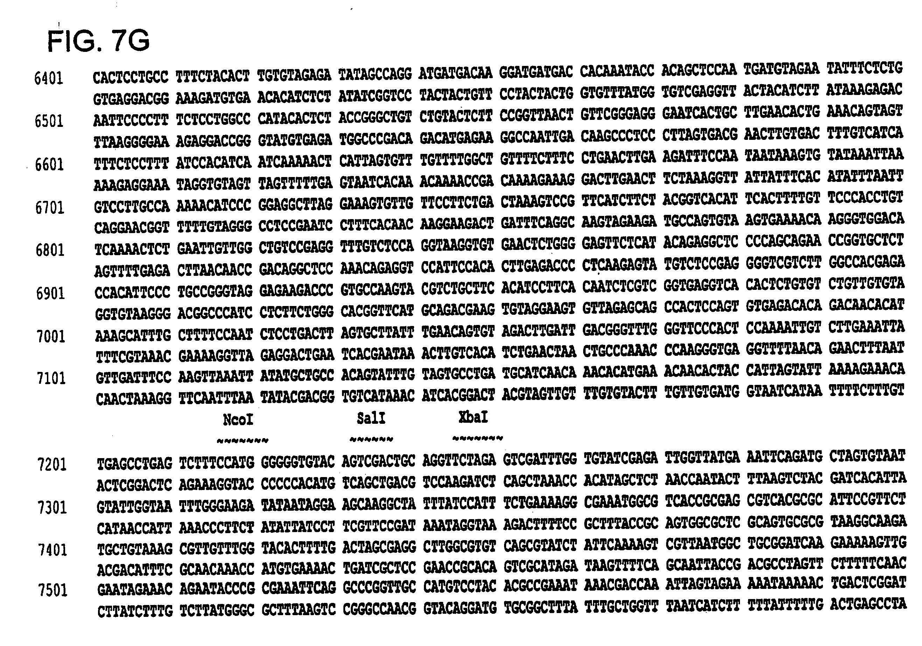

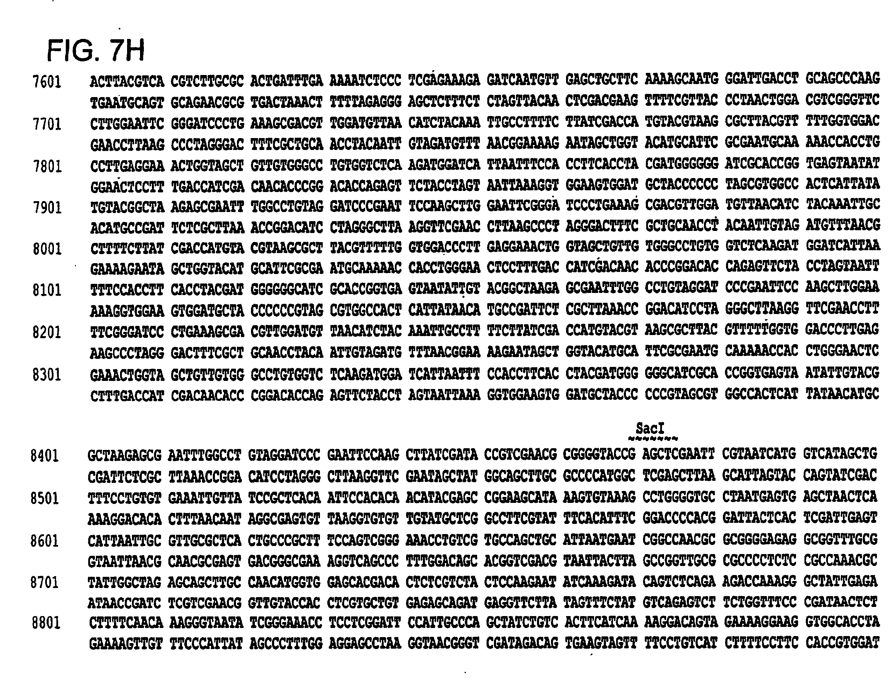

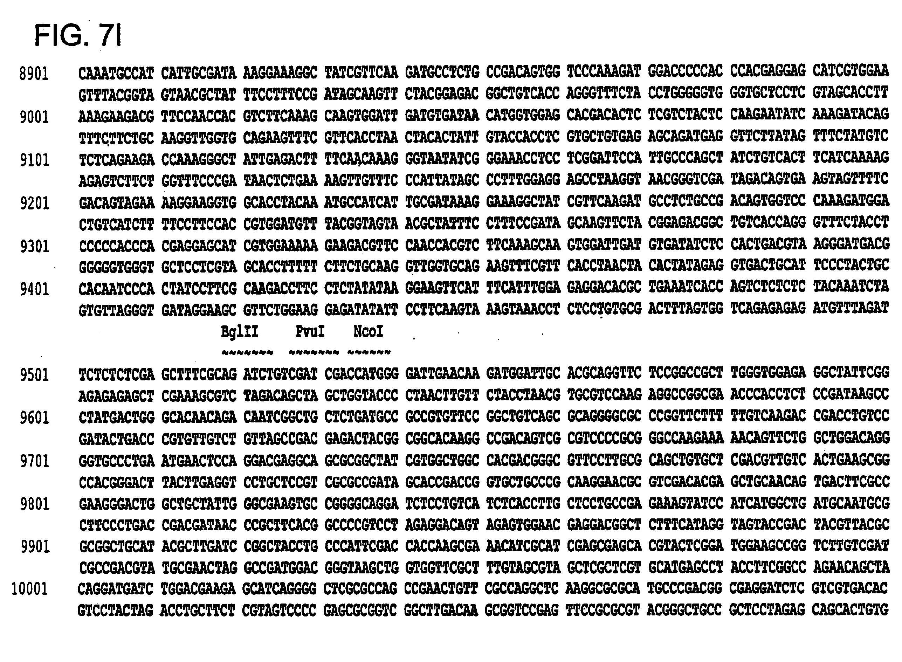

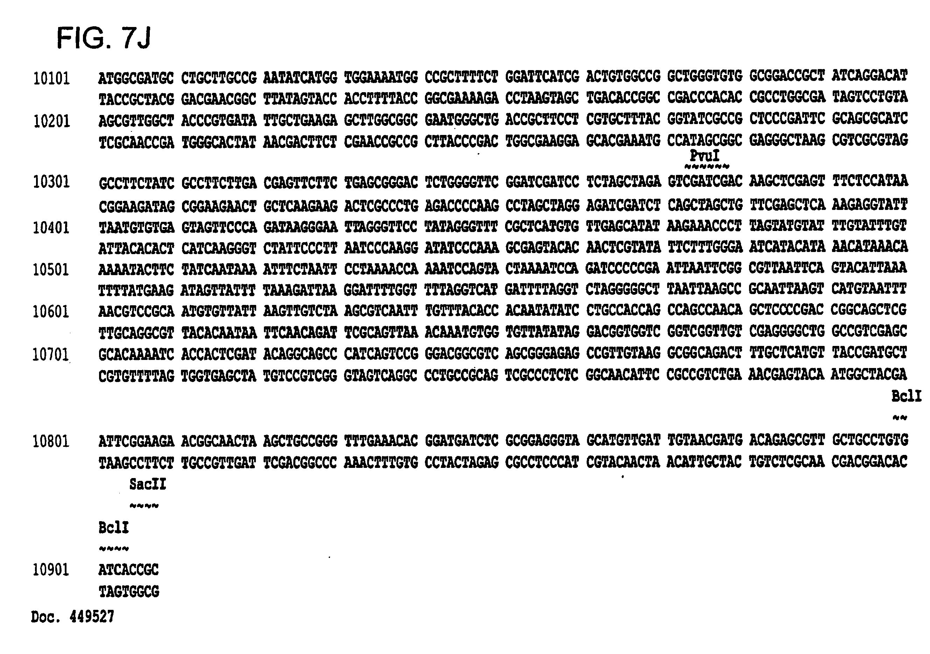

To construct the modified binary vector, the rhTF gene was copied from a plasmid containing the rhTF gene (or from a human cDNA) by polymerase chain reaction (PCR). Using PCR approach, the rhTF gene was fused to the tomato subtilase apoplast-targeting signal peptide (Janzik et al., 2000). A SAlI site was introduced by PCR at the 5'-end of the PCR product and an SpeI site was introduced at the 3'-end. To transfer the modified rhTF sequence into plant binary vector, a vector pSUNP2, bearing a kappa light chain antibody (see US 2004148656) was cleaved with SalI and SpeI and ligated to the rhTF PCR product cleaved with the same endonucleases. Other binary plant transformation vectors could also have been used (for example, pBIN19 (Bevan, 1984), which was an precursor of pSUNP2, or pBIN20 (Hennegan and Danna, 1998). In replacing the SalI-SpeI fragment containing the kappa light chain with SalI-SpeI fragment containing the rhTF fused to the subtilase signal, the coding region was inserted between (OCS)3Mas promoter and Nos-3' terminator. The resulting plasmid with rhTF was named pSUNP12 (a map is provided in Fig. 2) and a complete sequence of the vector is provided in Figs. 7A-F. For transient expression of rhTF in lettuce the, the pSUNP12 vector was transferred into *Agrobacterium tumefaciens* strain C58 (Sciaky et al., 1978) or any related strains (for example, C58C1).

### EXAMPLE 2: Preparation of Agrobacterium tumefaciens Suspension

*Agrobacterium tumefaciens* strain C58/C1 carrying the pSUNP12 vector were grown and vacuum-infiltrated into leaves of lettuce according to the procedure of transient expression in lettuce (USSN 10/739,447).

The *Agrobacterium* culture with the pSUNP12 binary vector was grown on LB broth supplemented with 5 g/L sucrose, 2 mM MgSO₄, 10 mM MES pH 5.6 and 20 µM acetosyringone overnight at 29°C. The ratio of the inoculum used to start the overnight culture to final culture volume was 1:100 and gave a suspension with a final OD₆₀₀ of about 2.4. The suspension was supplemented with 10 mM MES, pH 5.6, and 200 µM acetosyringone and incubated for 1 hour at 22°C. Before infiltration, 100 µM of 2,4-D and 0.005% Tween 20 were added. Lettuce was vacuum-infiltrated with the *Agrobacterium* culture and the leaves were treated as described in USSN 10/739,447 and Example 3. Lettuce leaves, transiently expressing rhTF, were used for isolation and purification of rhTF or alternatively, for preparation of crude extracts.

### EXAMPLE 3: Transformation of Lettuce

### Vacuum-infiltration and incubation of treated lettuce heads

1.25 L of pretreated suspension of *Agrobacterium* was placed into 2 L glass beaker inside vacuum-dessicator. Whole heads of lettuce were immersed into suspension and held for 20 min under a vacuum (25" column of water) and then, the vacuum was released rapidly. Lettuce heads were briefly rinsed in water and left for 3-4 days at 22°C and 16 hours of daylight in boxes with transparent cover on wet paper towels. After incubation, the treated lettuce was homogenized for protein extraction then used for purification of the rhTF as described in Example 4.

### EXAMPLE 4: Preparation of plant rhTF samples

Plant produced rhTF samples were prepared in purified form and as crude extract as follows:
1. Purification of lettuce produced rhTF: Leaf biomass was homogenized for 1 min at 4°C in Waring blender at maximum speed, with 1:1 of extraction buffer (100 mM Tris-HCl, pH8.0; 1 mM EDTA; 0.15 M NaCl; 1% Triton X-100). Homogenate was centrifuged for 15 min at 7,000 x g, then, supernatant was centrifuged for 15 min at 15,000 x g. Final supernatant was frozen and stored at - 70°C.

The frozen lettuce TF extraction was thawed at 4°C, and was centrifuged for 30 min at 20,000x g, followed by filtering the supernatant through 1 µm filter. Q Sepharose Fast Flow resin was equilibrated with 5 volumes of buffer (20 mM Tris-HCl, 0.1 M NaCl, 0.5% Tween 80, pH 7.5), then the supernatant was loaded onto the Q Sepharose resin and the unbound material was collected by vacuum. The Q flow-through was centrifuged at 20,000x g for 30 min, and the supernatant loaded onto an I47 affinity column pre-equilibrated with buffer (20 mM Tris-HCl, 0.1 M NaCl, 0.5% Tween 80, pH 7.5). After loading, the column was washed with 5 volumes of I47 column wash buffer 1 (20 mM Tris-HCl, 1 M NaCl, 0.5% glucoside, pH 8.0), buffer II (50 mM Na-phosphate, pH 6.0, 0.5% glucoside) and buffer III (100 mM Na-acetate, pH 4.0, 0.5% glucoside, 0.15 M NaCl), respectively, followed by eluting the column with 5 volumes of elution buffer (100 mM acetic acid, pH 3.0, 1% glucoside, 0.15 M NaCl). The protein peak was collected and adjusted to pH to 8.0 with 1 M Tris. The resulting TF was concentrate and buffer exchanged into storage buffer (20 mM Tris-HCl, 200 mM NaCl, pH 8.0, 1% glucoside). TF concentration was determined by A₂₈₀ using molar extinction coefficient of 44410 M⁻¹ (one OD₂₈₀ is equivalent to 0.67 mg/mL). For storage, it was frozen at -70°C.
2. Preparation of crude extract: Lettuce leaf was simply homogenized with an equal volume of PBS and centrifuged at 4°C for 5 min at 1000 x g. Supernatant was used as the crude extract. For storage it was frozen and kept at - 70°C.

### EXAMPLE 5: Characterization of purified plant rhTF

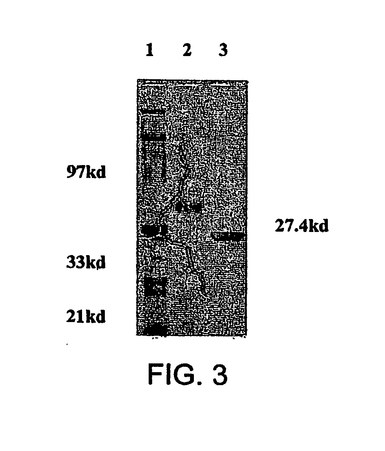

Purified lettuce TF was loaded on 12% NuPAGE Bis-Tris Gel to compare with *E. coli* produced rhTF₂₄₃ (Fig. 3). *E. coli* produced rhTF₂₄₃ molecular weight is 27.4 KD. Based on amino acid composition of lettuce TF₂₆₃, its theoretical molecular weight is 29.59 KD, however, we estimated by molecular weight markers on the gel that it's molecular mass is 35.4 KD indicating lettuce TF₂₆₃ is a glycosylated protein.

### A: Prothrombin time (PT) assay

Lipidated rhTF and crude TF extract were diluted with PT buffer (3 8 mM Tris-HCl, pH 7.5, 0.1% BSA, and 14.6 mM CaCl₂) to a series dilution. PT assays were initiated by injecting 0.2 mL of lipidated rhTF or crude lettuce TF extraction into plastic twin-well cuvettes. The cuvettes each contained 0.1 mL of the plasma pre-incubated with 0.01 mL of PT buffer for 1-2 minutes. When testing anti-TF antibody effect on lettuce TF, 0.01 mL of PT buffer was replaced by cH36. An automated coagulation timer (MLA Electra 800) was used to monitor Clot time.

### B: Comparative PT assay of purified E. coli rhTF(243aa), purified plant rhTF (full size - 263 aa), and plant rhTF crude extract

Purified lettuce rhTF and *E. coli* produced rhTF₂₄₃ (final concentration 1000 nM) were lipidated in a buffer containing 50 mM Tris-HCl, pH 7.5, 0.1% BSA, 14.6 mM CaCl₂, 0.07 mg/ml of phosphatidylcholine, and 0.03 mg/mL of phosphatidylserine at 37°C for 30 min. Then, lipidated TF was diluted at concentrations of 3, 1, 0.5, 0.25, 0.1, 0.05 and 0.025 nM in PT assay buffer (38 mM Tris-HCl, pH 7.5, 0.1 % BSA, 14.6 mM CaCl₂). Crude lettuce rhTF extract was quantified by ELISA, then diluted into the same scale as purified rhTF.

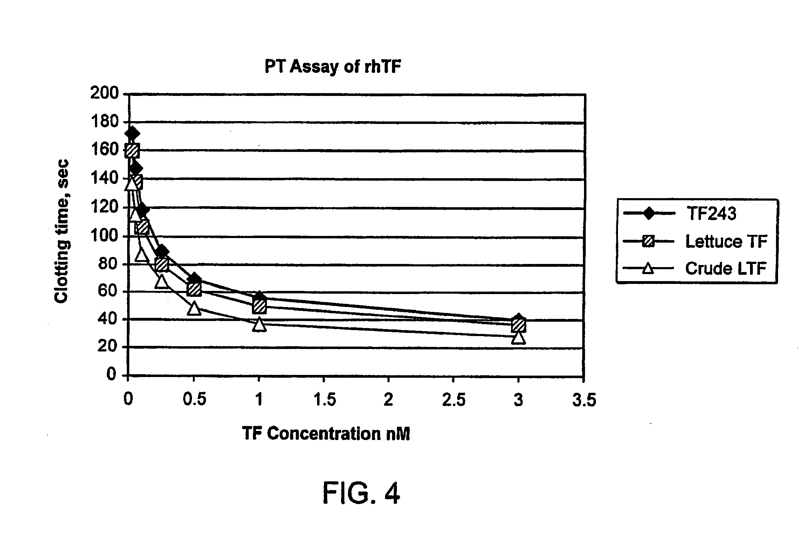

PT test was performed for these three samples. Fig. 4 shows that purified lettuce rhTF had similar clotting time as TF₂₄₃ indicating that lettuce produced rhTF is functioning, and its potency was comparable to the potency of *E. coli* TF₂₄₃. On the other hand, crude rhTF extract was compared with purified forms. The Fig. 4 shows that the potency of crude rhTF extract is similar as both purified forms, and pattern of dose response from three samples are same.

### C: Anti-TF monoclonal antibody effect on plant rhTF

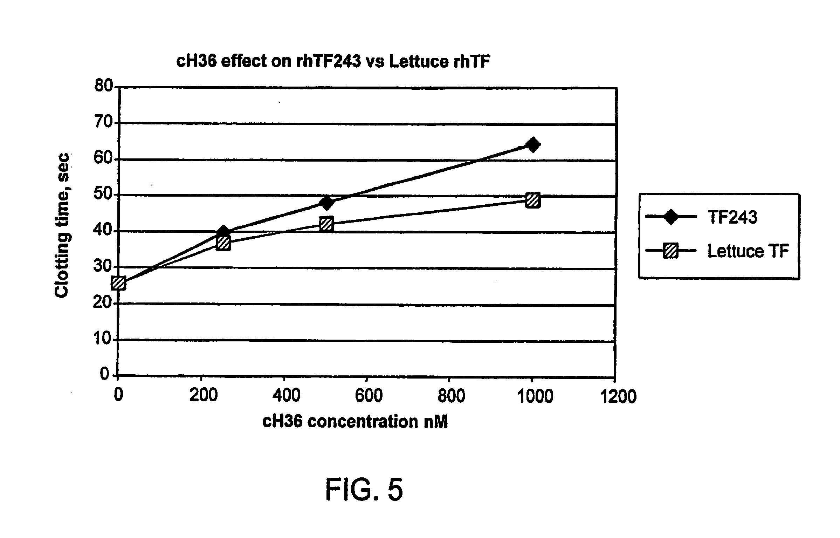

Anti-TF antibody (Sunol-cH36) effects on lettuce-produced rhTF was tested by PT assay. The antibody concentration was prepared as 0, 250 nM, 500 nM and 1000 nM. 10 µL of the antibody from each concentration was mixed and incubated with 100 µL of human plasma, respectively, then 200 µL of purified and lipidated rhTF at a concentration of 10 nM was added against different cH36 concentration. Fig. 5 indicates that anti-TF antibody, Sunol-cH36, does prolonged clotting time for lettuce-produced rhTF. For example, at antibody concentration of 500 nM, clotting time was increased from 25 sec to 42 sec, while compare to the effect to rhTF₂₄₃, which clotting time increased from 25 sec to 48 sec. Sunol-cH36, an anti-TF antibody, worked on both rhTF₂₄₃ and plant-produced rhTF₂₆₃. However, we noticed that at higher concentration of Sunol-cH36, it has less effect on lettuce-produced rhTF compared to the *E. coli* rhTF₂₄₃.

### EXAMPLE 6: Effect of plant rhTF on hemostasis in partial thickness wounds in a porcine model

The objective of this study was to examine the effect of a topical agent on hemostasis in partial thickness wounds in a porcine model.

### A. Experimental Animal

A pig was employed as the subject in this wound model due to the morphological similarities between swine skin and human skin. One young female specific pathogen free (SPF: Ken-O-Kaw Farms, Windsor, IL) pig weighing ∼50 kg was used. The animal was kept in house for at least two weeks prior to initiating the experiment and was fed a basal diet *ad libitum* and was housed individually in an approved animal facility (meeting American Association for Accreditation of Laboratory Animal Care [AAALAC] compliance) with controlled temperature (19-21°C) and lighting (12 hours with lighting /12 hours in darkness).

### B. Wounding Technique

The flank and back of the experimental animal was clipped with standard animal clippers on the day of the experiment. The skin on both sides of each animal was prepared for wounding by washing with a non-antibiotic soap (Neutrogena Soap Bar; Johnson and Johnson, Los Angeles, CA) and sterile water. Each animal was anesthetized intramuscularly with tiletamine HCl plus zolazepam (1.4 mg/kg) (Telazol; Laderle Parenterals Inc, Carolina, Puerto Rico), xylazine (2.0 mg/kg) (X-jet; Phoenix Scientific Inc, St. Joseph, MO), and atropine (0.04 mg/kg) (Atrojet SA; Phoenix Scientific Inc, St. Joseph, MO) followed by mask inhalation of an isoflurane (Isothesia; Abbott Laboratories, Chicago, IL) and oxygen combination.

Approximately fifty-two (52) rectangular wounds measuring 10 mm x 7 mm x 0.4 mm deep were made in the paravertebral and thoracic area with a specialized electrokeratome fitted with a 7 mm blade. The wounds were separated from one another by approximately 15 mm of unwounded skin.

### C. Treatments

Eight wounds were randomly assigned to each treatment group. Here we present results for 5 treatment groups:
1. Sterile water
2. PBS
*3. E. coli* rhTF₂₄₃ (100nM)
4. Purified plant rhTF (10nM
5. Crude extract of plant rhTF (10nM)

Samples were applied as a 0.2 mL volume, and were provided as materials in solution ready for application. Before experiment the specimens were placed in a 35°C water bath and kept in the water bath until treatment. All treatments were applied within five (5) minutes of wounding, and were visually inspected for hemostasis. Evaluation of hemostasis was made by measuring the time between application of the treatment and cessation of bleeding. Each wound received its own score; the eight scores from each treatment group are averaged, and those averages are graphed (see Table 2). Representative wounds from each treatment group were photographed.

Wounds were created and blotted after commencement of punctuate bleeding. All treatments were applied immediately after wounding and blotting, and were visually inspected for hemostasis. After each wound was created, blotted and the treatment applied, the effect of the treatment was observed for time to coagulation and for the extent of bleeding at the wound site. The treatments were only applied one time.

To determine the time to coagulation, a stopwatch was started when treatment was applied and stopped when it was determined that bleeding had ceased. This time was recorded. To determine the extent of bleeding, blood accumulation at the wound site was assessed according the following scale:
**Score: 1 = absent, 2 = mild, 3 = moderate, 4 = marked, 5 = considerable**
   **Note: a score of 3 was considered normal bleeding (*i.e.* untreated wound).** Representative wounds from each treatment group were photographed.

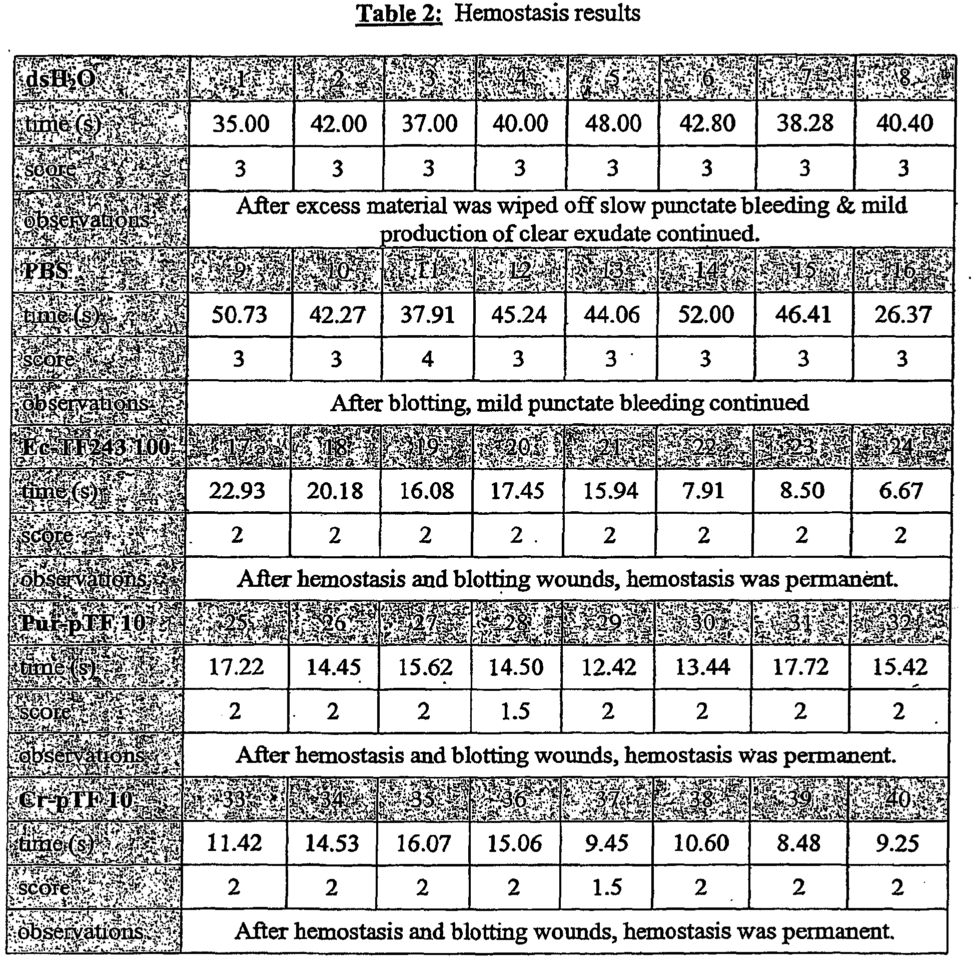

### D. Time to Hemostasis

Wounds treated with the dsH2O and PBS controls had an average time to hemostasis of 40 and 43 seconds, respectively. Wounds treated with Ec-TF243 100, Pur-pTF10, Cr-pTF10 had an average time to hemostasis of 14, 15, and 11 seconds, respectively.

### E. Score of Hemostasis

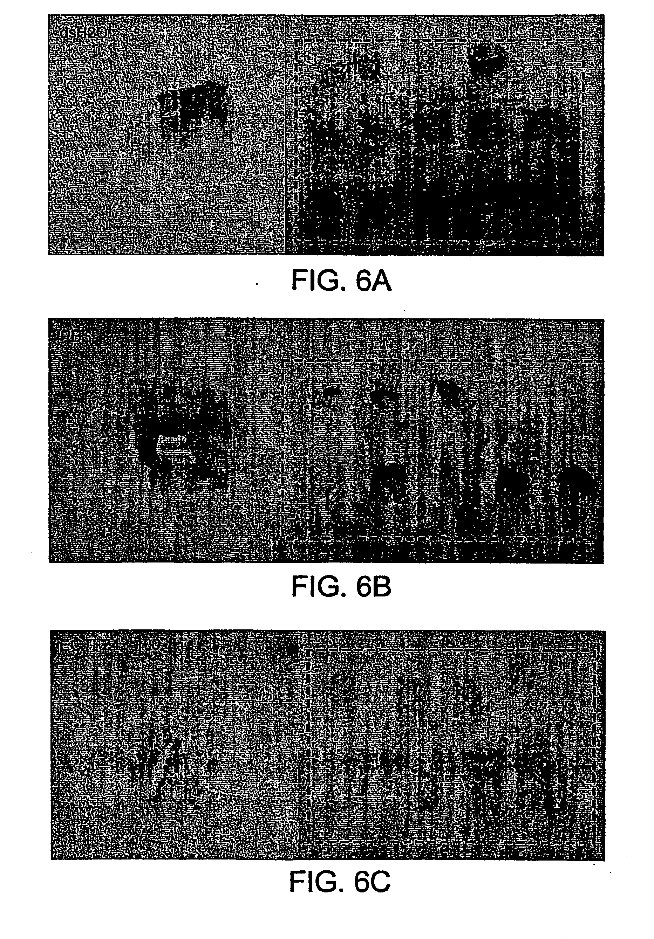

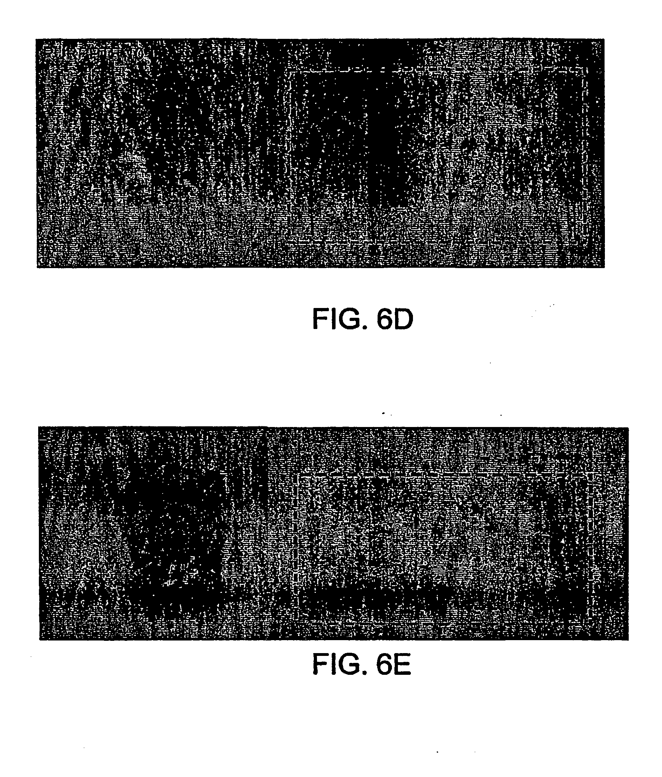

Wounds treated with the dsH₂O or PBS controls had average hemostasis scores of 3.00 and 3.12, respectively. Wounds treated with Ec-TF243 100, Pur-pTF10, or Cr-pTF10 had an average hemostasis score of 1.93, 2.00 and 1.93, respectively. Thus, Ec-TF243 100, Purp-pTF 10, and Cr-pTF 10 were able to maintain hemostasis, but both the untreated and PBS treated groups continued to bleed slowly (Fig. 6).

### Example 7: Stable Transgenic Plants Producing rhTF (referential)

Stable transgenic *Arabidopsis thaliana* (var. Columbia) plants were generated using *in planta* transformation by infiltration with *Agrobacterium* containing the pSUNP12 vector or a similar plant expression vector, pSUNP14. The pSUNP14 vector contains the rhTF gene fragment encoding amino acids 1 to 243 (i.e. terminating after the ... A I S L H sequence shown in Figure 1) instead for the rhTF₂₆₃ gene fragment of pSUNTP12. Plants were transformed as describes above, but the agro-infiltration was performed on flowering plants, a process referred to as floral dip (see for example, Clough SJ and Bent AF. 1998. The Plant Journal 16:735-743). After a month and a half, the plants produced T0 seeds. Seed germination and growth on solid media containing kanamycin (Km) resulted in the selection of T0 transgenic plants. Healthy green T0 seedlings were transferred into soil to yield over 100 transgenic plants with different levels of rhTF expression. The best candidates were selected for T1 seed production. On these, line A12-9 produced 4.3 mg/kg of rhTF₂₆₃ without induction, and line A14-17 produced 2.9 mg/kg of rhTF₂₄₃ without induction. Recombinant hTF expression can be further increased through induction of the (OCS)3MAS promoter by watering plants with 1 mM salicylic acid or by treating leaves with 2,4-D as described above. Transgenic *Arabidopsis* plants could be induced to produce at least 20 mg/kg of rhTF by these methods. T1 seeds from these plants will be used for further screening and generation of T2 plants.

### SEQUENCE LISTING

<110> ALTOR BIOSCIENCE CORPORATION
<120> PRODUCTION OF TISSUE FACTOR IN PLANTS
<130> 17264EP
<140> 05 762 794.5
   <141> 2005-06-22
<150> US 60/583,187
   <151> 2004-06-25
<160> 5
<170> PatentIn Ver. 3.3
<210> 1
   <211> 295
   <212> PRT
   <213> Homo sapiens
<400> 1
   Met Glu Thr Pro Ala Trp Pro Arg Val Pro Arg Pro Glu Thr Ala Val
   1 5 10 15
   Ala Arg Thr Leu Leu Leu Gly Trp Val Phe Ala Gln Val Ala Gly Ala
   20 25 30
   Ser Gly Thr Thr Asn Thr Val Ala Ala Tyr Asn Leu Thr Trp Lys Ser
   35 40 45
   Thr Asn Phe Lys Thr Ile Leu Glu Trp Glu Pro Lys Pro Val Asn Gln
   50 55 60
   Val Tyr Thr Val Gln Ile Ser Thr Lys Ser Gly Asp Trp Lys Ser Lys
   65 70 75 80
   Cys Phe Tyr Thr Thr Asp Thr Glu Cys Asp Leu Thr Asp Glu Ile Val
   85 90 95
   Lys Asp Val Lys Gln Thr Tyr Leu Ala Arg Val Phe Ser Tyr Pro Ala
   100 105 110
   Gly Asn Val Glu Ser Thr Gly Ser Ala Gly Glu Pro Leu Tyr Glu Asn
   115 120 125
   Ser Pro Glu Phe Thr Pro Tyr Leu Glu Thr Asn Leu Gly Gln Pro Thr
   130 135 140
   Ile Gln Ser Phe Glu Gln Val Gly Thr Lys Val Asn Val Thr Val Glu
   145 150 155 160
   Asp Glu Arg Thr Leu Val Arg Arg Asn Asn Thr Phe Leu Ser Leu Arg
   165 170 175
   Asp Val Phe Gly Lys Asp Leu Ile Tyr Thr Leu Tyr Tyr Trp Lys Ser
   180 185 190
   Ser Ser Ser Gly Lys Lys Thr Ala Lys Thr Asn Thr Asn Glu Phe Leu
   195 200 205
   Ile Asp Val Asp Lys Gly Glu Asn Tyr Cys Phe Ser Val Gln Ala Val
   210 215 220
   Ile Pro Ser Arg Thr Val Asn Arg Lys Ser Thr Asp Ser Pro Val Glu
   225 230 235 240
   Cys Met Gly Gln Glu Lys Gly Glu Phe Arg Glu Ile Phe Tyr Ile Ile
   245 250 255
   Gly Ala Val Val Phe Val Val Ile Ile Leu Val Ile Ile Leu Ala Ile
   260 265 270
   Ser Leu His Lys Cys Arg Lys Ala Gly Val Gly Gln Ser Trp Lys Glu
   275 280 285
   Asn Ser Pro Leu Asn Val Ser
   290 295
<210> 2
   <211> 286
   <212> PRT
   <213> Homo sapiens
<400> 2
   Met Glu Arg Leu Arg Leu Met Phe Leu Leu Ile Leu Met Val Val Leu
    1               5                  10                  15
   Phe His Val Phe Val Asp Ala Ser Gly Thr Thr Asn Thr Val Ala Ala
               20                  25                  30
   Tyr Asn Leu Thr Trp Lys Ser Thr Asn Phe Lys Thr Ile Leu Glu Trp
           35                  40                  45
   Glu Pro Lys Pro Val Asn Gln Val Tyr Thr Val Gln Ile Ser Thr Lys
       50                  55                  60
   Ser Gly Asp Trp Lys Ser Lys Cys Phe Tyr Thr Thr Asp Thr Glu Cys
   65                  70                  75                  80
   Asp Leu Thr Asp Glu Ile Val Lys Asp Val Lys Gln Thr Tyr Leu Ala
                   85                  90                  95
   Arg Val Phe Ser Tyr Pro Ala Gly Asn Val Glu Ser Thr Gly Ser Ala
               100                 105                 110
   Gly Glu Pro Leu Tyr Glu Asn Ser Pro Glu Phe Thr Pro Tyr Leu Glu
           115                 120                 125
   Thr Asn Leu Gly Gln Pro Thr Ile Gln Ser Phe Glu Gln Val Gly Thr
       130                 135                 140
   Lys Val Asn Val Thr Val Glu Asp Glu Arg Thr Leu Val Arg Arg Asn
   145                 150                 155                 160
   Asn Thr Phe Leu Ser Leu Arg Asp Val Phe Gly Lys Asp Leu Ile Tyr
                   165                 170                 175
   Thr Leu Tyr Tyr Trp Lys Ser Ser Ser Ser Gly Lys Lys Thr Ala Lys
               180                 185                 190
   Thr Asn Thr Asn Glu Phe Leu Ile Asp Val Asp Lys Gly Glu Asn Tyr
           195                 200                 205
   Cys Phe Ser Val Gln Ala Val Ile Pro Ser Arg Thr Val Asn Arg Lys
       210                 215                 220
   Ser Thr Asp Ser Pro Val Glu Cys Met Gly Gln Glu Lys Gly Glu Phe
   225                 230                 235                 240
   Arg Glu Ile Phe Tyr Ile Ile Gly Ala Val Val Phe Val Val Ile Ile
                   245                 250                 255
   Leu Val Ile Ile Leu Ala Ile Ser Leu His Lys Cys Arg Lys Ala Gly
               260                 265                 270
   Val Gly Gln Ser Trp Lys Glu Asn Ser Pro Leu Asn Val Ser
           275                 280                 285
<210> 3
   <211> 10808
   <212> DNA
   <213> Artificial Sequence
<220>
   <223> Description of Artificial Sequence: Synthetic nucleotide construct
<400> 3
   ggtttcaaaa tcggctccgt cgatactatg ttatacgcca actttgaaaa caactttgaa 60
   aaagctgttt tctggtattt aaggttttag aatgcaagga acagtgaatt ggagttcgtc 120
   ttgttataat tagcttcttg gggtatcttt aaatactgta gaaaagagga aggaaataat 180
   aaatggctaa aatgagaata tcaccggaat tgaaaaaact gatcgaaaaa taccgctgcg 240
   taaaagatac ggaaggaatg tctcctgcta aggtatataa gctggtggga gaaaatgaaa 300
   acctatattt aaaaatgacg gacagccggt ataaagggac cacctatgat gtggaacggg 360
   aaaaggacat gatgctatgg ctggaaggaa agctgcctgt tccaaaggtc ctgcactttg 420
   aacggcatga tggctggagc aatctgctca tgagtgaggc cgatggcgtc ctttgctcgg 480
   aagagtatga agatgaacaa agccctgaaa agattatcga gctgtatgcg gagtgcatca 540
   ggctctttca ctccatcgac atatcggatt gtccctatac gaatagctta gacagccgct 600
   tagccgaatt ggattactta ctgaataacg atctggccga tgtggattgc gaaaactggg 660
   aagaagacac tccatttaaa gatccgcgcg agctgtatga ttttttaaag acggaaaagc 720
   ccgaagagga acttgtcttt tcccacggcg acctgggaga cagcaacatc tttgtgaaag 780
   atggcaaagt aagtggcttt attgatcttg ggagaagcgg cagggcggac aagtggtatg 840
   acattgcctt ctgcgtccgg tcgatcaggg aggatatcgg ggaagaacag tatgtcgagc 900
   tattttttga cttactgggg atcaagcctg attgggagaa aataaaatat tatattttac 960
   tggatgaatt gttttagtac ctagaatgca tgaccaaaat cccttaacgt gagttttcgt 1020
   tccactgagc gtcagacccc gtagaaaaga tcaaaggatc ttcttgagat cctttttttc 1080
   tgcgcgtaat ctgctgcttg caaacaaaaa aaccaccgct accagcggtg gtttgtttgc 1140
   cggatcaaga gctaccaact ctttttccga aggtaactgg cttcagcaga gcgcagatac 1200
   caaatactgt ccttctagtg tagccgtagt taggccacca cttcaagaac tctgtagcac 1260
   cgcctacata cctcgctctg ctaatcctgt taccagtggc tgctgccagt ggcgataagt 1320
   cgtgtcttac cgggttggac tcaagacgat agttaccgga taaggcgcag cggtcgggct 1380
   gaacgggggg ttcgtgcaca cagcccagct tggagcgaac gacctacacc gaactgagat 1440
   acctacagcg tgagctatga gaaagcgcca cgcttcccga agggagaaag gcggacaggt 1500
   atccggtaag cggcagggtc ggaacaggag agcgcacgag ggagcttcca gggggaaacg 1560
   cctggtatct ttatagtcct gtcgggtttc gccacctctg acttgagcgt cgatttttgt 1620
   gatgctcgtc aggggggcgg agcctatgga aaaacgccag caacgcggcc tttttacggt 1680
   tcctggcctt ttgctggcct tttgctcaca tgttctttcc tgcgttatcc cctgattctg 1740
   tggataaccg tattaccgcc tttgagtgag ctgataccgc tcgccgcagc cgaacgaccg 1800
   agcgcagcga gtcagtgagc gaggaagcgg aagagcgcct gatgcggtat tttctcctta 1860
   cgcatctgtg cggtatttca caccgcatat ggtgcactct cagtacaatc tgctctgatg 1920
   ccgcatagtt aagccagtat acactccgct atcgctacgt gactgggtca tggctgcgcc 1980
   ccgacacccg ccaacacccg ctgacgcgcc ctgacgggct tgtctgctcc cggcatccgc 2040
   ttacagacaa gctgtgaccg tctccgggag ctgcatgtgt cagaggtttt caccgtcatc 2100
   accgaaacgc gcgaggcagg gtgccttgat gtgggcgccg gcggtcgagt ggcgacggcg 2160
   cggcttgtcc gcgccctggt agattgcctg gccgtaggcc agccattttt gagcggccag 2220
   cggccgcgat aggccgacgc gaagcggcgg ggcgtaggga gcgcagcgac cgaagggtag 2280
   gcgctttttg cagctcttcg gctgtgcgct ggccagacag ttatgcacag gccaggcggg 2340
   ttttaagagt tttaataagt tttaaagagt tttaggcgga aaaatcgcct tttttctctt 2400
   ttatatcagt cacttacatg tgtgaccggt tcccaatgta cggctttggg ttcccaatgt 2460
   acgggttccg gttcccaatg tacggctttg ggttcccaat gtacgtgcta tccacaggaa 2520
   agagaccttt tcgacctttt tcccctgcta gggcaatttg ccctagcatc tgctccgtac 2580
   attaggaacc ggcggatgct tcgccctcga tcaggttgcg gtagcgcatg actaggatcg 2640
   ggccagcctg ccccgcctcc tccttcaaat cgtactccgg caggtcattt gacccgatca 2700
   gcttgcgcac ggtgaaacag aacttcttga actctccggc gctgccactg cgttcgtaga 2760
   tcgtcttgaa caaccatctg gcttctgcct tgcctgcggc gcggcgtgcc aggcggtaga 2820
   gaaaacggcc gatgccggga tcgatcaaaa agtaatcggg gtgaaccgtc agcacgtccg 2880
   ggttcttgcc ttctgtgatc tcgcggtaca tccaatcagc tagctcgatc tcgatgtact 2940
   ccggccgccc ggtttcgctc tttacgatct tgtagcggct aatcaaggct tcaccctcgg 3000
   ataccgtcac caggcggccg ttcttggcct tcttcgtacg ctgcatggca acgtgcgtgg 3060
   tgtttaaccg aatgcaggtt tctaccaggt cgtctttctg ctttccgcca tcggctcgcc 3120
   ggcagaactt gagtacgtcc gcaacgtgtg gacggaacac gcggccgggc ttgtctccct 3180
   tcccttcccg gtatcggttc atggattcgg ttagatggga aaccgccatc agtaccaggt 3240
   cgtaatccca cacactggcc atgccggccg gccctgcgga aacctctacg tgcccgtctg 3300
   gaagctcgta gcggatcacc tcgccagctc gtcggtcacg cttcgacaga cggaaaacgg 3360
   ccacgtccat gatgctgcga ctatcgcggg tgcccacgtc atagagcatc ggaacgaaaa 3420
   aatctggttg ctcgtcgccc ttgggcggct tcctaatcga cggcgcaccg gctgccggcg 3480
   gttgccggga ttctttgcgg attcgatcag cggccgcttg ccacgattca ccggggcgtg 3540
   cttctgcctc gatgcgttgc cgctgggcgg cctgcgcggc cttcaacttc tccaccaggt 3600
   catcacccag cgccgcgccg atttgtaccg ggccggatgg tttgcgaccg tcacgccgat 3660
   tcctcgggct tgggggttcc agtgccattg cagggccggc agacaaccca gccgcttacg 3720
   cctggccaac cgcccgttcc tccacacatg gggcattcca cggcgtcggt gcctggttgt 3780
   tcttgatttt ccatgccgcc tcctttagcc gctaaaattc atctactcat ttattcattt 3840
   gctcatttac tctggtagct gcgcgatgta ttcagatagc agctcggtaa tggtcttgcc 3900
   ttggcgtacc gcgtacatct tcagcttggt gtgatcctcc gccggcaact gaaagttgac 3960
   ccgcttcatg gctggcgtgt ctgccaggct ggccaacgtt gcagccttgc tgctgcgtgc 4020
   gctcggacgg ccggcactta gcgtgtttgt gcttttgctc attttctctt tacctcatta 4080
   actcaaatga gttttgattt aatttcagcg gccagcgcct ggacctcgcg ggcagcgtcg 4140
   ccctcgggtt ctgattcaag aacggttgtg ccggcggcgg cagtgcctgg gtagctcacg 4200
   cgctgcgtga tacgggactc aagaatgggc agctcgtacc cggccagcgc ctcggcaacc 4260
   tcaccgccga tgcgcgtgcc tttgatcgcc cgcgacacga caaaggccgc ttgtagcctt 4320
   ccatccgtga cctcaatgcg ctgcttaacc agctccacca ggtcggcggt ggcccatatg 4380
   tcgtaagggc ttggctgcac cggaatcagc acgaagtcgg ctgccttgat cgcggacaca 4440
   gccaagtccg ccgcctgggg cgctccgtcg atcactacga agtcgcgccg gccgatggcc 4500
   ttcacgtcgc ggtcaatcgt cgggcggtcg atgccgacaa cggttagcgg ttgatcttcc 4560
   cgcacggccg cccaatcgcg ggcactgccc tggggatcgg aatcgactaa cagaacatcg 4620
   gccccggcga gttgcagggc gcgggctaga tgggttgcga tggtcgtctt gcctgacccg 4680
   cctttctggt taagtacagc gataaccttc atgcgttccc cttgcgtatt tgtttattta 4740
   ctcatcgcat catatacgca gcgaccgcat gacgcaagct gttttactca aatacacatc 4800
   acctttttag acggcggcgc tcggtttctt cagcggccaa gctggccggc caggccgcca 4860
   gcttggcatc agacaaaccg gccaggattt catgcagccg cacggttgag acgtgcgcgg 4920
   gcggctcgaa cacgtacccg gccgcgatca tctccgcctc gatctcttcg gtaatgaaaa 4980
   acggttcgtc ctggccgtcc tggtgcggtt tcatgcttgt tcctcttggc gttcattctc 5040
   ggcggccgcc agggcgtcgg cctcggtcaa tgcgtcctca cggaaggcac cgcgccgcct 5100
   ggcctcggtg ggcgtcactt cctcgctgcg ctcaagtgcg cggtacaggg tcgagcgatg 5160
   cacgccaagc agtgcagccg cctctttcac ggtgcggcct tcctggtcga tcagctcgcg 5220
   ggcgtgcgcg atctgtgccg gggtgagggt agggcggggg ccaaacttca cgcctcgggc 5280
   cttggcggcc tcgcgcccgc tccgggtgcg gtcgatgatt agggaacgct cgaactcggc 5340
   aatgccggcg aacacggtca acaccatgcg gccggccggc gtggtggtgt cggcccacgg 5400
   ctctgccagg ctacgcaggc ccgcgccggc ctcctggatg cgctcggcaa tgtccagtag 5460
   gtcgcgggtg ctgcgggcca ggcggtctag cctggtcact gtcacaacgt cgccagggcg 5520
   taggtggtca agcatcctgg ccagctccgg gcggtcgcgc ctggtgccgg tgatcttctc 5580
   ggaaaacagc ttggtgcagc cggccgcgtg cagttcggcc cgttggttgg tcaagtcctg 5640
   gtcgtcggtg ctgacgcggg catagcccag caggccagcg gcggcgctct tgttcatggc 5700
   gtaatgtctc cggttctagt cgcaagtatt ctactttatg cgactaaaac acgcgacaag 5760
   aaaacgccag gaaaagggca gggcggcagc ctgtcgcgta acttaggact tgtgcgacat 5820
   gtcgttttca gaagacggct gcactgaacg tcagaagccg actgcactat agcagcggag 5880
   gggttggatc aaagtacttt gatcccgagg ggaaccctgt ggttggcatg cacatacaaa 5940
   tggacgaacg gataaacctt ttcacgccct tttaaatatc cgttattcta ataaacgctc 6000
   ttttctctta ggtttacccg ccaatatatc ctgtcaaaca ctgatagttt aaactcatgt 6060
   ttgacagctt atcatcggat ctagtaacat agatgacacc gcgcgcgata atttatccta 6120
   gtttgcgcgc tatattttgt tttctatcgc gtattaaatg tataattgcg ggactctaat 6180
   cataaaaacc catctcataa ataacgtcat gcattacatg ttaattatta catgcttaac 6240
   gtaattcaac agaaattata tgataatcat cgcaagaccg gcaacaggat tcaatcttaa 6300
   gaaactttat tgccaaatgt ttgaacgatc ggggccggcc agggatctcc ggactagttt 6360
   atgaaacatt cagtggggag ttctccttcc agctctgccc cactcctgcc tttctacact 6420
   tgtgtagaga tatagccagg atgatgacaa ggatgatgac cacaaatacc acagctccaa 6480
   tgatgtagaa tatttctctg aattcccctt tctcctggcc catacactct accgggctgt 6540
   ctgtactctt ccggttaact gttcgggagg gaatcactgc ttgaacactg aaacagtagt 6600
   tttctccttt atccacatca atcaaaaact cattagtgtt tgttttggct gttttctttc 6660
   ctgaacttga agatttccaa taataaagtg tataaattaa gtccttgcca aaaacatccc 6720
   ggaggcttag gaaagtgttg ttccttctga ctaaagtccg ttcatcttct acggtcacat 6780
   tcacttttgt tcccacctgt tcaaaactct gaattgttgg ctgtccgagg tttgtctcca 6840
   ggtaaggtgt gaactctggg gagttctcat acagaggctc cccagcagaa ccggtgctct 6900
   ccacattccc tgccgggtag gagaagaccc gtgccaagta cgtctgcttc acatccttca 6960
   caatctcgtc ggtgaggtca cactctgtgt ctgttgtgta aaagcatttg cttttccaat 7020
   ctcctgactt agtgcttatt tgaacagtgt agacttgatt gacgggtttg ggttcccact 7080
   ccaaaattgt cttgaaatta gttgatttcc aagttaaatt atatgctgcc acagtatttg 7140
   tagtgcctga tgcatcaaca aacacatgaa acaacactac cattagtatt aaaagaaaca 7200
   tgagcctgag tctttccatg gggggtgtac agtcgactgc aggttctaga gtcgatttgg 7260
   tgtatcgaga ttggttatga aattcagatg ctagtgtaat gtattggtaa tttgggaaga 7320
   tataatagga agcaaggcta tttatccatt tctgaaaagg cgaaatggcg tcaccgcgag 7380
   cgtcacgcgc attccgttct tgctgtaaag cgttgtttgg tacacttttg actagcgagg 7440
   cttggcgtgt cagcgtatct attcaaaagt cgttaatggc tgcggatcaa gaaaaagttg 7500
   gaatagaaac agaatacccg cgaaattcag gcccggttgc catgtcctac acgccgaaat 7560
   aaacgaccaa attagtagaa aaataaaaac tgactcggat acttacgtca cgtcttgcgc 7620
   actgatttga aaaatctccc tcgagaaaga gatcaatgtt gagctgcttc aaaagcaatg 7680
   ggattgacct gcagcccaag cttggaattc gggatccctg aaagcgacgt tggatgttaa 7740
   catctacaaa ttgccttttc ttatcgacca tgtacgtaag cgcttacgtt tttggtggac 7800
   ccttgaggaa actggtagct gttgtgggcc tgtggtctca agatggatca ttaatttcca 7860
   ccttcaccta cgatgggggg atcgcaccgg tgagtaatat tgtacggcta agagcgaatt 7920
   tggcctgtag gatcccgaat tccaagcttg gaattcggga tccctgaaag cgacgttgga 7980
   tgttaacatc tacaaattgc cttttcttat cgaccatgta cgtaagcgct tacgtttttg 8040
   gtggaccctt gaggaaactg gtagctgttg tgggcctgtg gtctcaagat ggatcattaa 8100
   tttccacctt cacctacgat ggggggcatc gcaccggtga gtaatattgt acggctaaga 8160
   gcgaatttgg cctgtaggat cccgaattcc aagcttggaa ttcgggatcc ctgaaagcga 8220
   cgttggatgt taacatctac aaattgcctt ttcttatcga ccatgtacgt aagcgcttac 8280
   gtttttggtg gacccttgag gaaactggta gctgttgtgg gcctgtggtc tcaagatgga 8340
   tcattaattt ccaccttcac ctacgatggg gggcatcgca ccggtgagta atattgtacg 8400
   gctaagagcg aatttggcct gtaggatccc gaattccaag cttatcgata ccgtcgaacg 8460
   cggggtaccg agctcgaatt cgtaatcatg gtcatagctg tttcctgtgt gaaattgtta 8520
   tccgctcaca attccacaca acatacgagc cggaagcata aagtgtaaag cctggggtgc 8580
   ctaatgagtg agctaactca cattaattgc gttgcgctca ctgcccgctt tccagtcggg 8640
   aaacctgtcg tgccagctgc attaatgaat cggccaacgc gcggggagag gcggtttgcg 8700
   tattggctag agcagcttgc caacatggtg gagcacgaca ctctcgtcta ctccaagaat 8760
   atcaaagata cagtctcaga agaccaaagg gctattgaga cttttcaaca aagggtaata 8820
   tcgggaaacc tcctcggatt ccattgccca gctatctgtc acttcatcaa aaggacagta 8880
   gaaaaggaag gtggcaccta caaatgccat cattgcgata aaggaaaggc tatcgttcaa 8940
   gatgcctctg ccgacagtgg tcccaaagat ggacccccac ccacgaggag catcgtggaa 9000
   aaagaagacg ttccaaccac gtcttcaaag caagtggatt gatgtgataa catggtggag 9060
   cacgacactc tcgtctactc caagaatatc aaagatacag tctcagaaga ccaaagggct 9120
   attgagactt ttcaacaaag ggtaatatcg ggaaacctcc tcggattcca ttgcccagct 9180
   atctgtcact tcatcaaaag gacagtagaa aaggaaggtg gcacctacaa atgccatcat 9240
   tgcgataaag gaaaggctat cgttcaagat gcctctgccg acagtggtcc caaagatgga 9300
   cccccaccca cgaggagcat cgtggaaaaa gaagacgttc caaccacgtc ttcaaagcaa 9360
   gtggattgat gtgatatctc cactgacgta agggatgacg cacaatccca ctatccttcg 9420
   caagaccttc ctctatataa ggaagttcat ttcatttgga gaggacacgc tgaaatcacc 9480
   agtctctctc tacaaatcta tctctctcga gctttcgcag atctgtcgat cgaccatggg 9540
   gattgaacaa gatggattgc acgcaggttc tccggccgct tgggtggaga ggctattcgg 9600
   ctatgactgg gcacaacaga caatcggctg ctctgatgcc gccgtgttcc ggctgtcagc 9660
   gcaggggcgc ccggttcttt ttgtcaagac cgacctgtcc ggtgccctga atgaactcca 9720
   ggacgaggca gcgcggctat cgtggctggc cacgacgggc gttccttgcg cagctgtgct 9780
   cgacgttgtc actgaagcgg gaagggactg gctgctattg ggcgaagtgc cggggcagga 9840
   tctcctgtca tctcaccttg ctcctgccga gaaagtatcc atcatggctg atgcaatgcg 9900
   caggatgatc tggacgaaga gcatcagggg ctcgcgccag ccgaactgtt cgccaggctc 9960
   aaggcgcgca tgcccgacgg cgaggatctc gtcgtgacac atggcgatgc ctgcttgccg 10020
   aatatcatgg tggaaaatgg ccgcttttct ggattcatcg actgtggccg gctgggtgtg 10080
   gcggaccgct atcaggacat agcgttggct acccgtgata ttgctgaaga gcttggcggc 10140
   gaatgggctg accgcttcct cgtgctttac ggtatcgccg ctcccgattc gcagcgcatc 10200
   gccttctatc gccttcttga cgagttcttc tgagcgggac tctggggttc ggatcgatcc 10260
   tctagctaga gtcgatcgac aagctcgagt ttctccataa taatgtgtga gtagttccca 10320
   gataagggaa ttagggttcc tatagggttt cgctcatgtg ttgagcatat aagaaaccct 10380
   tagtatgtat ttgtatttgt aaaatacttc tatcaataaa atttctaatt cctaaaacca 10440
   aaatccagta ctaaaatcca gatcccccga attaattcgg cgttaattca gtacattaaa 10500
   aacgtccgca atgtgttatt aagttgtcta agcgtcaatt tgtttacacc acaatatatc 10560
   ctgccaccag ccagccaaca gctccccgac cggcagctcg gcacaaaatc accactcgat 10620
   acaggcagcc catcagtccg ggacggcgtc agcgggagag ccgttgtaag gcggcagact 10680
   ttgctcatgt taccgatgct attcggaaga acggcaacta agctgccggg tttgaaacac 10740
   ggatgatctc gcggagggta gcatgttgat tgtaacgatg acagagcgtt gctgcctgtg 10800
   atcaccgc                                                          10808
<210> 4
   <211> 263
   <212> PRT
   <213> Homo sapiens
<400> 4
   Ser Gly Thr Thr Asn Thr Val Ala Ala Tyr Asn Leu Thr Trp Lys Ser
    1               5                  10                  15
   Thr Asn Phe Lys Thr Ile Leu Glu Trp Glu Pro Lys Pro Val Asn Gln
               20                  25                  30
   Val Tyr Thr Val Gln Ile Ser Thr Lys Ser Gly Asp Trp Lys Ser Lys
           35                  40                  45
   Cys Phe Tyr Thr Thr Asp Thr Glu Cys Asp Leu Thr Asp Glu Ile Val
       50                  55                  60
   Lys Asp Val Lys Gln Thr Tyr Leu Ala Arg Val Phe Ser Tyr Pro Ala
   65                  70                  75                  80
   Gly Asn Val Glu Ser Thr Gly Ser Ala Gly Glu Pro Leu Tyr Glu Asn
                   85                  90                  95
   Ser Pro Glu Phe Thr Pro Tyr Leu Glu Thr Asn Leu Gly Gln Pro Thr
               100                 105                 110
   Ile Gln Ser Phe Glu Gln Val Gly Thr Lys Val Asn Val Thr Val Glu
           115                 120                 125
   Asp Glu Arg Thr Leu Val Arg Arg Asn Asn Thr Phe Leu Ser Leu Arg
       130                 135                 140
   Asp Val Phe Gly Lys Asp Leu Ile Tyr Thr Leu Tyr Tyr Trp Lys Ser
   145                 150                 155                 160
   Ser Ser Ser Gly Lys Lys Thr Ala Lys Thr Asn Thr Asn Glu Phe Leu
                   165                 170                 175
   Ile Asp Val Asp Lys Gly Glu Asn Tyr Cys Phe Ser Val Gln Ala Val
               180                 185                 190
   Ile Pro Ser Arg Thr Val Asn Arg Lys Ser Thr Asp Ser Pro Val Glu
           195                 200                 205
   Cys Met Gly Gln Glu Lys Gly Glu Phe Arg Glu Ile Phe Tyr Ile Ile
       210                 215                 220
   Gly Ala Val Val Phe Val Val Ile Ile Leu Val Ile Ile Leu Ala Ile
   225                 230                 235                 240
   Ser Leu His Lys Cys Arg Lys Ala Gly Val Gly Gln Ser Trp Lys Glu
                   245                 250                 255
   Asn Ser Pro Leu Asn Val Ser
               260
<210> 5
   <211> 32
   <212> PRT
   <213> Homo sapiens

## Claims

1. A recombinant mammalian tissue factor obtained from a transgenic lettuce (*Lactucua sativa*) and comprising at least one plant-specific glycan wherein the recombinant mammalian tissue factor can be used as a coagulant without significant artificial lipidation, and wherein the plant-specific glycan lacks at least one and preferably both of terminal galactose and terminal acetylneuraminic acid (NeuAc; sialic acid) group and includes at least one of and preferably both of α(1,3) fucose and β(1,2) xylose group.

2. The recombinant mammalian tissue factor of claim 1, wherein the plant-specific glycan includes an α(1,3) fucose and a β (1;2) xylose group.

3. The recombinant mammalian tissue factor of one of claims 1 or 2, wherein the α(1,3) fucose is covalently linked to a N-acetylglucosamine (GlcNAc) group.

4. The recombinant mammalian tissue factor of one of claims 1 to 3, for use in the prevention or treatment of blood loss in a subject.

5. The recombinant mammalian tissue factor for the use of claim 4, wherein the subject is a human patient in need of treatment.

6. The recombinant mammalian tissue factor for the use of claim 4, wherein the subject is a mammal in need of treatment.

7. The recombinant mammalian tissue factor of one of claims 1 to 3, for use in the promotion of wound healing in a subject.

8. The recombinant mammalian tissue factor of one of claims 1 to 3, for use in the promotion of angiogenesis in a subject.

9. The recombinant mammalian tissue factor of one of claims 1 to 3, for use in the promotion of vascular remodelling in a subject.

10. An absorbent comprising the recombinant mammalian tissue factor of one of claims 1 to 3.

11. The absorbent of claim 10, wherein the absorbent is medical gauze.

12. The absorbent of claim 11, wherein the medical gauze comprises cotton fibers or a fiber blend comprising cotton.

13. The absorbent of one of claims 10 to 12, wherein the absorbent comprises a pharmaceutically acceptable solution.

14. An adhesive support comprising the absorbent of one of claims 10 to 13.

15. The adhesive support of claim 14, wherein the adhesive support is sterile.

16. A wrapper container comprising the adhesive support of claim 14, wherein the wrapper container is essentially impervious to water and air.

## Patentansprüche

1. Rekombinanter Säugergewebefaktor, der aus einem transgenen Lattich *(Lactuca sativa)* erhalten wird und wenigstens ein pflanzenspezifisches Glycan umfasst, wobei der rekombinante Säugergewebefaktor als Koagulans ohne signifikante künstliche Lipidierung verwendet werden kann und wobei dem pflanzenspezifischen Glycan wenigstens eine und vorzugsweise beide der terminalen Galactose- und terminalen Acetylneuraminsäure (NeuAc; Sialinsäure)-Gruppen fehlt/fehlen und das pflanzenspezifische Glyan wenigstens eine und vorzugsweise beide der α(1,3)-Fucose- und β(1,2)-Xylose-Gruppen umfasst.

2. Rekombinanter Säugergewebefaktor gemäß Anspruch 1, wobei das pflanzenspezifische Glycan eine α(1,3)-Fucose- und eine β(1,2)-Xylose-Gruppe umfasst.

3. Rekombinanter Säugergewebefaktor gemäß einem der Ansprüche 1 oder 2, wobei die α(1,3)-Fucose kovalent an eine N-Acetylglucosamin(GlcNAc)-Gruppe gebunden ist.

4. Rekombinanter Säugergewebefaktor gemäß einem der Ansprüche 1 bis 3 zur Verwendung bei der Prävention oder Behandlung von Blutverlust bei einem Subjekt.

5. Rekombinanter Säugergewebefaktor zur Verwendung gemäß Anspruch 4, wobei das Subjekt ein menschlicher Patient ist, der Bedarf für eine Behandlung hat.

6. Rekombinanter Säugergewebefaktor zur Verwendung gemäß Anspruch 4, wobei das Subjekt ein Säuger ist, der Bedarf für eine Behandlung hat.

7. Rekombinanter Säugergewebefaktor gemäß einem der Ansprüche 1 bis 3 zur Verwendung bei der Begünstigung der Wundheilung bei einem Subjekt.

8. Rekombinanter Säugergewebefaktor gemäß einem der Ansprüche 1 bis 3 zur Verwendung bei der Begünstigung von Angiogenese bei einem Subjekt.

9. Rekombinanter Säugergewebefaktor gemäß einem der Ansprüche 1 bis 3 zur Verwendung bei der Begünstigung der Gefäßnachbildung bei einem Subjekt.

10. Absorbens, das den rekombinanten Säugergewebefaktor gemäß einem der Ansprüche 1 bis 3 umfasst.

11. Absorbens gemäß Anspruch 10, wobei das Absorbens medizinische Gaze ist.

12. Absorbens gemäß Anspruch 11, wobei die medizinische Gaze Baumwollfasern oder eine Fasermischung, die Baumwolle umfasst, umfasst.

13. Absorbens gemäß einem der Ansprüche 10 bis 12, wobei das Absorbens eine pharmazeutisch verträgliche Lösung umfasst.

14. Klebstoffträger, der das Absorbens gemäß einem der Ansprüche 10 bis 13 umfasst.

15. Klebstoffträger gemäß Anspruch 14, wobei der Klebstoffträger steril ist.

16. Verpackungsbehälter, umfassend den Klebstoffträger gemäß Anspruch 1, wobei der Verpackungsbehälter gegenüber Wasser und Luft im Wesentlichen unempfindlich ist.

## Revendications

1. Un facteur tissulaire recombinant de mammifère obtenu d'une laitue transgénique (Lactuca sativa) et comprenant au moins un glycane spécifique aux plantes, le facteur tissulaire recombinant de mammifère pouvant être utilisé comme un anticoagulant sans une lipidation artificielle importante, et dans lequel le glycane spécifique aux plantes manque au moins l'un et de préférence les deux des groupes: le groupe galactose terminal et le groupe terminal d'acide acétylneuraminique (NeAc; acide sialique), et comprend au moins l'un et de préférence les deux des groupes de α(1,3) fucose et de β(1,2) xylose.

2. Le facteur tissulaire recombinant de mammifère selon la revendication 1, dans lequel le glycane spécifique aux plantes comprend un groupe de α(1,3) fucose et un groupe de β(1,2) xylose.

3. Le facteur tissulaire recombinant selon l'une quelconque des revendications 1 et 2, dans lequel le α(1,3) fucose est lié de manière covalente à un groupe N-acetylglucosamine (GlcNAc).

4. Le facteur tissulaire recombinant de mammifère selon l'une quelconque des revendications 1 à 3 pour l'utilisation dans la prévention ou le traitement de la perte de sang chez un sujet.

5. Le facteur tissulaire recombinant de mammifère pour l'utilisation selon la revendication 4, le sujet étant un patient humain ayant besoin d'un traitement.

6. Le facteur tissulaire recombinant de mammifère pour l'utilisation selon la revendication 4, le sujet étant un mammifère ayant besoin d'un traitement.

7. Le facteur tissulaire recombinant de mammifère selon l'une quelconque des revendications 1 à 3 pour l'utilisation dans la stimulation de la cicatrisation chez un sujet.

8. Le facteur tissulaire recombinant de mammifère selon l'une quelconque des revendications 1 à 3 pour l'utilisation dans la stimulation de l'angiogenèse chez un sujet.

9. Le facteur tissulaire recombinant de mammifère selon l'une quelconque des revendications 1 à 3 pour l'utilisation dans la stimulation du remodelage vasculaire.

10. Un absorbant comprenant le facteur tissulaire recombinant de mammifère selon l'une quelconque des revendications 1 à 3.

11. L'absorbant selon la revendication 10, dans lequel l'absorbant est une gaze médicale.

12. L'absorbant selon la revendication 11, la gaze médicale comprenant des fibres de coton ou un mélange de fibres comprenant du coton.

13. L'absorbant selon l'une quelconque des revendications 10 à 12, dans lequel l'absorbant comprend une solution pharmaceutiquement acceptable.

14. Un support adhésif comprenant l'absorbant selon l'une quelconque des revendications 10 à 13.

15. Le support adhésif selon la revendication 14, le support adhésif étant stérile.

16. Un récipient enveloppe comprenant le support adhésif selon la revendication 14, le récipient enveloppe étant essentiellement imperméable à l'eau et à l'air.
